# EUROPEAN PATENT APPLICATION

(11) **EP 1 674 478 A1**
(43) Date of publication of application: **28.06.2006**
(21) Application number: 04030473.5
(22) Date of filing: 22.12.2004
(51) Int. Cl.: C07K 14/47, G01N 33/68, G01N 33/58, C12N 15/00

(54) **Fusion proteins and method for determining protein-protein-interactions in living cells and cell lysates, nucleic acids encoding these fusion proteins, as well as vectors and kits containing these**

(71) Applicant: GSF-Forschungszentrum für Umwelt und Gesundheit GmbH, 85764 Oberschleissheim (DE)
(72) Inventor: Wolff, Horst, Dr., 81677 München (DE); Brebeck, Andrea, 85635 Höhenkirchen-Siegertsbrunn (DE); Ziegler, Manja, 80797 München (DE); Brack-Werner, Ruth, Dr., 80999 München (DE)
(74) Representative: Graf von Stosch, Andreas

(57) **Abstract**

The present invention refers to inventive fusion proteins, to a method for detecting protein-protein-interactions in living cells and cell lysates using these inventive fusion proteins. The present invention also refers to a screening method for identifying compounds suitable to modify, i.e. inhibit or enhance, protein-protein-interaction using these fusion proteins. Additionally a method for detecting cells is disclosed comprising an unknown protein that interacts with a known protein. Along with the inventive fusion proteins used for these methods encoding nucleic acids, corresponding vectors and host cells transfected accordingly are disclosed herewith.

## Description

The present invention refers to inventive fusion proteins, to a method for detecting protein-protein-interactions in living cells and cell lysates using these inventive fusion proteins. The present invention also refers to a screening method for identifying compounds suitable to modify, i.e. inhibit or enhance, protein-protein-interaction using these fusion proteins. Additionally a method for detecting cells is disclosed comprising an unknown protein that interacts with a known protein. Along with the inventive fusion proteins used for these methods encoding nucleic acids, corresponding vectors and host cells transfected accordingly are disclosed herewith.

The term "protein-protein-interactions" according to the present invention refers to the property of protein molecules to bind to each other such as to form a complex. Protein-protein-interactions are involved in many biological processes, including e.g. signal transduction pathways, enzyme-substrate interactions, viral adhesions and formation of antibody-antigen-complexes, etc.. Identifying and characterizing such interactions is fundamental for understanding biological mechanisms, as well as for characterising the molecular basis of various diseases, e.g. HIV (see e.g. Gallo et al. (1983), Science 220(4599)865-7; and Barre-Sinoussi et al. (1983), Science 220(4599):868-71).

Detailed knowledge about molecular processes, such as the molecular mechanism of infections, penetration of viruses into the cells, reproduction of viruses on a molecular level, etc. allows to outline strategies for treating diseases, e.g. viral infectious diseases such as AIDS, SARS, influenza etc. By way of example detailed knowledge about interactions of (surface) proteins or virus-associated factors with their physiological (host cell) binding partners is essential in order to decipher the mechanisms of infection and to elucidate suitable therapies. Protein-protein-interactions of mechanistic relevance are of particular interest in this context.

Various systems for determining protein-protein-interactions are known in the art. Typically, protein-protein-interactions are determined by established biochemical (separation) methods, such as affinity chromatography, Western Blots or co-immunoprecipitation. By these techniques, molecular properties of proteins may be determined. However, these methods may not be applied to the analysis of protein-protein-interactions in living cells. Accordingly, no information may be gathered with these state-of-the-art methods about protein-protein-interactions in a cellular environment, in particular in living cells. Furthermore, biochemical methods as listed above are frequently associated with purifying large amounts of protein to be tested or by expensive preparation of suitable antibodies.

Two-hybrid-systems allow to determine protein-protein-interactions in a cellular environment. Methods based on two-hybrid-systems were initially developed for determining protein-protein-interactions in yeast cells and were adapted subsequently for use in mammalian or prokaryotic cells. In the yeast-2-hybrid (Y2H) system the transcriptional activator protein Gal4 is split into two portions (domains), its DNA-binding domain and its activator domain. Assembling both domains allows to restore the biological function of the corresponding full-length protein. In order to determine protein-protein interactions in a cellular environment both domains are encoded on two different vectors, whereby each of the domains is provided as part of a fusion gene. One fusion gene contains a gene for a DNA-binding domain, e.g. the Gal4 DNA-binding domain, and a gene encoding e.g. a protein to be tested (protein I). The other fusion gene encodes a DNA-activator domain, e.g. the Gal4 activator domain, and the potential binding ligand (protein II) of the protein to be tested. Protein I is termed "bait" and protein II is termed "prey", the potential interaction of which with protein I is to be tested. Fusion proteins containing "prey" and "bait" are expressed in the same host cell after transfection of these vectors. If prey and bait portions of fusion proteins interact with each other, both domains of Gal4 assemble to form a functional Gal4 activator protein, thereby activating the expression of a reporter gene, such as the LacZ-gene, in the host cell. Consequently, an interaction of the "prey" and "bait" portions of both fusion proteins can be identified phenotypically, e.g. by blue colonies.

However, the Y2H-system is typically suitable for the investigation of yeast proteins and other proteins, which do not require a specific cellular environment for e.g. posttranslational modifications. Some other proteins cannot be examined in the Y2H-system because of their specific (e.g. mammalian) cell prerequisites. In order to overcome these problems, alternatives to the Y2H system have been developed, e.g. the prokaryotic-two-hybrid (P2H)-system or the mammalian-two-hybrid (M2H)-system. These systems follow the same general principle as outlined for Y2H.

In the P2H-system, e.g. as disclosed in WO 98/25947, prokaryotic host cells are used, such as E. coli, Salmonella, Pseudomonas, etc.. The P2H-system provides fusion proteins to be tested and reporter genes, typically reporter genes suitable for prokaryotic cell systems. According to the teaching of WO 98/25947 both potentially interacting fusion proteins comprise either a DNA-binding domain or a DNA-interacting domain. Upon interacting of both fusion proteins a functional activating protein is formed, which initiates an altered expression of a reporter gene as a detectable signal.

In the M2H-system, mammalian cells are used as host cells. These mammalian cells allow posttranslational modifications to be introduced into the proteins to be tested as eventually required for the biological function of mammalian proteins. E.g. misfolding of mammalian proteins, as regularly observed in other non-mammalian host cell systems due to the absence of posttranslational modifications, may be avoided in the M2H-system. Interaction of proteins in the M2H-system is determined in a similar way as in the Y2H-system. In the M2H-system, one fusion protein contains a DNA binding-domain, e.g. the DNA-binding domain of Gal4, fused to a protein to be tested (protein I). The second fusion protein typically consists of a mammalian activating domain, e.g. the activating domain of mammalian NF-κB, fused to the potential binding ligand (protein II) of the protein to be tested. Any mammalian reporter gene as well as other reporters such as luciferase may be used for detection of functional activator protein activation. However, none of the above two-hybrid-systems allows to control the transfection efficiency. If host cells are not transfected by vectors (for whatever reason), false-negative signals are observed, leading to substantial uncertainty when interpreting the results. Furthermore, the underlying concept of the state-of-the-art two-hybrid-systems does not allow to localize the fusion proteins as such or as part of the interaction complex in the cells.

Another system, used for the determination of protein-protein-interactions in a cell, is FRET (fluorescence resonance energy transfer). The mechanism of the FRET-system is based on a non-radiating transfer of photonic energy of an activated fluorophore (donor) to a second fluorophore (acceptor), provided that both are located in close proximity (1-10 nm). If the donor and the acceptor are fused to two distinct protein domains, both the donor and the acceptor get into close proximity, if both protein domains interact with each other. As a result, a FRET effect may be observed. Acceptors and/or donors as used in the FRET-system are typically fluorochromes, e.g. GFP or derivates from GFP (Green fluorescent Protein), such as CFP and YFP. Their activation and emission spectra have been proved to be suitable for experiments based on a FRET system. However, the FRET system also bears some disadvantages. E.g. it has to be ensured that filters for detecting single spectra match exactly in order to avoid translucence of fluorescence from other channels. Furthermore, multiple analysis of the same sample is complicated seriously, since the FRET signal is weakened during analysis. This is due to bleaching of the donor upon activation during analysis under the microscope.

The so-called BiFC (Bimolecular Fluorescence Complementation)-system (see Hu et al., (2002), Molecular Cell, Vol. 9,789-798) represents an alternative to the FRET system, if protein-protein-interactions are to be analysed in the cell. The general concept behind the BiFC system is based on the interaction of portions of a fluorescent protein. More precisely, a signal is observed from both portions brought into proximity to each other, if proteins to be tested and fused to the fluorescent portions interact with each other. Since the analysis of protein-protein-interactions in cell lysates as well as in living cells is accessible by the BiFC technique, cellular mechanisms may be examined by this method. However, as with other prior art methods plasmid transfection cannot be tested by the BiFC system. This situation causes a major disadvantage of this method. Therefore, absence of fluorescence signals may be due to unsuccessful transfection. Additionally, localisation within the cell is limited to the finally established complex by its observable YFP signal. As a consequence, it remains unclear as to where the respective proteins are localised as such and as to whether the attached portions influence protein localisation. Normally, maturing of YFP in a cell (t_{1/2}) requires about 30 minutes (Hu et al. (2002), supra). Consequently, the interaction site of both proteins may differ significantly from the cellular location of the complex sending out the YFP signal and, therefore, may not be determined by the BiFC system.

Consequently, (i) the interaction of two proteins and (ii) their cell localisation cannot be simultaneously detected by using prior art methods.

It is the object of the present invention to provide a method for determining protein-protein-interactions qualitatively and quantitatively in cell lysates or in living cells. The method shall allow to simultaneously localize the distribution of the proteins to be tested in the cell. It is a further object of the present invention to provide a screening method for potential inhibitors and/or enhancers of protein-protein-interactions, also to be used in cell lysates or in living cells. Another object of the present invention is to provide a method enabling a person skilled in the art to screen cells for potentially interacting protein candidates.

The objects are solved by a fusion protein according to the present invention. The fusion protein according to the present invention contains the following components: (a) a protein sequence of a protein to be tested, (b) a fluorochrome group, and (c) the N-terminal or C-terminal protein portion of a fluorochrome protein, whereby the fluorochrome group (b) is preferably not essentially identical with the N-terminal or C-terminal portion of the fluorochrome protein (c).

Components (a), (b) and (c) as portions of the inventive fusion protein may be located in any conceivable order, e.g. a fusion protein according to the present invention may contain components (a), (b) and (c) in the order (a), (b), (c); or (c), (b), (a); or (a), (c), (b); or (b), (c), (a); or (b), (a), (c); or (c), (a), (b) (from N- to C-terminus). Preferably, components (a), (b) and (c) of the inventive fusion protein are provided as (a), (b), (c) or (c), (b), (a) (from N- to C-terminus).

The physiological protein or protein fragment to be tested, being present as component (a) of a fusion protein according to the present invention, may typically be any protein or protein fragment, the potential protein-protein interaction of which with any other protein is to be analysed. The protein to be tested, being component (a) of the fusion protein according to the present invention, is typically a prokaryotic or eukaryotic protein, preferably a viral, bacterial, plant, human or animal protein. It may be provided in its full length form or as partial sequence, e.g. domains. The protein to be tested may comprise for instance one or more interaction domains of a protein, such as the SH1, SH2 or SH3-domain of a protein to be tested, or, alternatively one or more binding domains or multimerisation domains. The protein to be tested is preferably derived from the family of transcription factors, growth factors, cell surface proteins, or may be an intracellular signal transduction protein, in particular a protein involved in transduction of G-protein coupled signals or a matrix or cellular skeletal protein. The protein to be tested alternatively may be derived from ubiquitine, ubiquitine related proteins or proteins sharing a significant similarity with ubiquitine (e.g. more than 75% sequence identity with the amino acid sequence of ubiquitine), or other proteins involved in intracellular decomposition processes, or fragments therof. Furthermore, the protein to be tested may be any non-cellular protein transfected into a host cell, or proteins interacting therewith. Insofar, transfected non-cellular proteins are preferably viral or bacterial proteins. Particularly preferred as component (a) of a fusion protein according to the present invention are viral proteins, e.g. proteins of the Myxo virus, Influenza virus, SARS (Severe Acute Respiratory Syndrome)-virus, proteins of Entero viruses, Orthomyxo viruses (Influenza viruses), Paramyxo viruses, Herpes-Simplex-virus, Adenovirus, Varizella-/Zoster-virus, Epstein-Barr-virus, Zytomegalie, Rubella virus, HIV virus, or host cell proteins exhibiting binding properties to these non-cellular proteins. Component (a) of a fusion protein of the present invention may also be selected from the groups consisting of bacterial proteins, in particular proteins from intracellularly parasitic bacterial species, such as chlamydia, TBC, etc.

A fluorochrome group (component (b)) of the fusion protein in terms of the present invention is meant to be any group which may be activiated such as to emit a fluorescence signal. "Fluorochrome" in the meaning of the present disclosure is thus equivalent with "fluorescent" or "capable of emitting a fluorescence signal". In the terms of the present invention a fluorochrome group of a fusion protein is preferably a linker of any chemical moiety having fluorescence properties, or a fluorescence dye coupled to a peptide or a protein, or an intrinsic fluorochrome protein. A fluorescence dye is typically any fluorescence dye capable of being coupled to a peptide or a protein, preferably via its side chains. Preferably, the fluorescence dye is selected from hemicyanes, e.g. DY-630-NHS or DY-635-NHS, or from coumarins, e.g. aminocoumarin, 7-amino-4-methylcoumarin, 7-hydroxy-4-methylcoumarin, AMCA (aminomethylcoumarin), 15, IAEDANS, hydroxycoumarin, etc..

Alternatively, any fluorochrome protein, which can be activated such as to emit fluorescence signal, can be used as component (b) of the inventive fusion protein as well. Preferably, the fluorochrome protein has no dimerizing properties in order to exclude any impairment of the protein-protein-interaction by fluorochrome protein dimerization. Preferably, the fluorochrome protein occurs in a monomeric form in the cell. The fluorochrome protein is typically selected from any fluorescent protein, e.g. from a group comprising the blue fluorescent protein (BFP), the green fluorescent protein (GFP), the photo activatable-GFP (PA-GFP), the yellow shifted green fluorescent protein (Yellow GFP), the yellow fluorescent protein (YFP), the enhanced yellow fluorescent protein (EYFP), the cyan fluorescent protein (CFP), the enhanced cyan fluorescent protein (ECFP), the monomeric red fluorescent protein (mRFP1), the kindling fluorescent protein (KFP1), aequorin, the autofluorescent proteins (AFPs), or the fluorescent proteins JRed, TurboGFP, PhiYFP and PhiYFP-m, tHc-Red (HcRed-Tandem), PS-CFP2 and KFP-Red (all available from EVRΩGEN, see also www.evrogen.com), or other suitable fluorescent proteins.

Component (c) of the fusion protein according to the present invention is a fluorochrome protein and may be selected from the group of fluorescent proteins as referenced above for component (b). For the fusion protein according to the present invention, component (c) shall not reflect the full-length sequence of a fluorochrome protein, but rather a portion of this full-length sequence. Typically, component (c) will either contain the N-terminal or the C-terminal portion of the native fluorochrome protein. Preferably, the N- or C-terminal portion of a fluorochrome protein (forming (or being part of) component (c)) will not emit fluorescent signals upon activation. However, it must be ensured that portions used as component (c) regain their fluorescent properties upon contact with one another. Typically, the portion of a fluorescent protein as used for component (c) comprises at least one domain of a full-length fluorochrome protein or any other structurally independent unit of the full-length fluorochrome protein. Component (c) is advantageously essentially not identical with the afore disclosed fluorochrome group of component (b). Not identical means that components (b) and (c) are preferably selected from e.g. different fluorochrome proteins. However, if component (c) and component (b) rely on the same fluorochrome protein, component (c) may represent a portion (e.g. a domain) of the full-length fluorochrome protein sequence of component (b). Alternatively, component (c) may represent an altered sequence of the non-altered fluorochrome protein sequence of component (b), or vice versa. If i.e. two fusion proteins according to the present invention, each having an N-terminal or a C-terminal portion of a fluorochrome protein as component (c), respectively, are brought together in a host cell and interact with each other via components (a) and (a'), components (c) of fusion protein A and A', etc. come into proximity and regain their fluorescent properties such as to emit a fluorescence signal upon activation.

Apart from the above mentioned components (a), (b) and (c), the inventive fusion protein may contain at least one linker, which spatially separates at least two of the afore disclosed components. Preferably, components (a) and (b) and/or (b) and (c) are separated by a linker. Typically, such a linker is an oligo- or polypeptide. Preferably, a linker has a length of 5-40 amino acids, more preferably a length of 5 to 25 amino acids and most preferably a length of 5 to 20 amino acids. Advantageously, the fusion protein according to the present invention comprises a linker without secondary structure forming properties, i.e. without α-helix or a β-sheet structure forming tendency. More preferably, the linker is composed of at least 35 % of glycin residues. Typically, a linker will be selected from the peptide sequences as given by SEQ ID NO:1 or SEQ ID NO:2.

The inventive fusion protein or rather its components may be labelled for further detection, e.g. component (a), i.e. the protein to be tested, component (b), i.e. the fluorochrome group, and/or component (c), i.e. the fluorochrome protein or additional linker sequences. A label is preferably selected from the group of labels comprising:
(i) radioactive labels, i.e. radioactive phosphorylation or a radioactive label with sulphur, hydrogen, carbon, nitrogen, etc.
(ii) coloured dyes (e.g. digoxygenin, etc.)
(iii) fluorescent groups (e.g. fluorescein, etc.)
(iv) chemoluminescent groups,
(v) groups for immobilisation on a solid phase (e.g. biotin, streptag, antibodies, antigene, etc.) and
(vi) a combination of labels of two or more of the labels mentioned under (i) to (v).

It may be advantageous to label the inventive fusion protein with more than one label for determining protein-protein-interactions. The chemical character of a label may depend on the specific experimental conditions. Cell lysate protein-protein-interaction experiments with inventive fusion proteins will allow to use any of the afore-mentioned labels . In contrast, in living cells testing will preferably be carried out with a non-bulky group and/or with a group exerting no deleterious effects to a cell.

According to the invention sets of fusion proteins A, A', etc. are provided. Fusion proteins A and A' contains different proteins (protein fragments) as component (a) and (a'), the interaction potential of which is to be analysed. In addition, these fusion proteins A and A', etc. preferably differ in their components (b). A fusion protein A may comprise as component (b) a fluorochrome group, being different from fluorochrome group (as component (b')) of a fusion protein A'). Finally, fusion proteins A and A' will typically differ as to their components (c). As explained above, fusion protein A will contain e.g. the N-terminal portion of a fluorochrome protein as component (c), whereas fusion protein A' will contain its C-terminal portion as component (c). Due to the different moieties used for components (b) and, typically, for components (c) several fluorescence signals may be observed, if fusion proteins A and A' have been transfected simultaneously and successfully into a host cell. If the set of fusion proteins A and A' interact via their components (a), a fluorescence signal may be detected due to the assembly of the fluorochrome protein portions of components (c) and (c'). Two further signals from each fusion protein A and A' are due to their fluorescent components (b) and (b'). In summary, just one fluorescence signal may be detected, if just one inventive fusion protein is successfully expressed in the host cells. At least two fluorescence signals (fluorescent signals of components (b) and b')) will be observed, if both inventive fusion proteins are expressed due to successful transfection of both underlying vectors. However, if these inventive fusion proteins A and A' interact via their components (a) and (a'), three fluorescence signals will be visible. Due to different fluorescence signals of components (b) and (b') of A and A' additional information about the protein-protein-interaction status is gained according to the invention. The absence of protein-protein-interactions due to technical problems (e.g. due to ineffective transfection of a vector, encoding the inventive fusion proteins, or due to deficient expression of fusion proteins A and A' in a host cell) allows just one signal to be observed. This experimental condition may be experimentally distinguished from the situation of non-existent protein-protein-interactions, even though both fusion proteins are successfully expressed by the host cells (two signals of components (b) and (b')).

Fusion proteins according to the present invention may also contain components (a), (b) and (c), which deviate in sequence from the native proteins, but are functionally homologous. Typically, such functionally homologous components (a), (b) and (c) of the inventive fusion proteins, encoded by inventive nucleic acid sequences as defined below, have at least 90 %, preferably at least 95 % sequence identity to their corresponding native parent proteins or protein fragments.

Another subject of the present invention refers to nucleic acids, encoding the inventive fusion protein. An inventive nucleic acid may be preferably a nucleic acid/nucleic acid sequence N, N', etc., encoding an inventive fusion protein A, A', etc., as defined above. A nucleic acid encoding component (a) of an inventive fusion protein within the meaning of the present invention may be encoded by genomic DNA, subgenomic DNA, cDNA, synthetic DNA, and/or combinations thereof. Additionally, an inventive nucleic acid is preferably selected from any nucleic sequence variant encoding the same amino acid sequence of an inventive fusion protein (due to degeneration of the genetic code). E.g. these alternative nucleic acid sequences may lead to an improved expression of the encoded fusion protein in a selected host organism. Tables for appropriately adjusting a nucleic acid sequence are known to a skilled person. Preparation and purification of such nucleic acids and/or derivatives are usually carried out by standard procedures (see Sambrook et al. 2001, Molecular Cloning: A Laboratory Manual, Cold Spring Harbor, NY). Other variants of native nucleic acid sequences for components (a), (b) and/or (c), which fall under the scope of the present invention, have one or more codon(s) inserted, deleted and/or substituted as compared to native nucleic acid sequences. These sequence variants for components (a), (b) and/or (c) lead to inventive fusion proteins having at least one amino acid substituted, deleted and/or inserted as compared to the native amino acid sequences. Therefore, nucleic acid sequences may code for modified (non-natural) components (a), components (b) and/or components (c) of inventive fusion proteins.

Another embodiment of the present invention refers to vectors V, in particular to expression vectors, containing at least one inventive nucleic acid sequence. A vector according to the present invention may be a vector V, V', etc., containing a nucleic acid N, N', etc., as defined above, encoding an inventive fusion protein A, A', etc.. These vectors V, V', etc. may preferably contain each a nucleic acid sequence N, N', etc., encoding an inventive fusion protein A, A', etc.. Thereby, each vector V, V', etc. contains only one inventive nucleic acid sequence N, N', etc.. Alternatively, a vector V according to the present invention contains more than one inventive nucleic acid N, N', etc.. If an inventive vector V contains more than one nucleic acid N, N', etc., these nucleic acids are preferably non-identical. Nucleic acids N and N' differ from each other by their components (a) and (a') and, preferably additional, by their components (b), (b') and/or (c), (c').

A "vector" within the meaning of the present invention advantageously comprises at least one inventive nucleic acid N, N', etc. and typically additional elements suitable for directing expression of the encoded inventive fusion proteins A, A', etc.. One class of vectors as used herein utilizes DNA elements that provide autonomously replicating extrachromosomal plasmids derived from animal viruses (e.g., bovine papilloma virus, polyomavirus, adenovirus, or SV40, etc.). A second class of inventive vectors as used herein relies upon the integration of the desired gene sequences into the host cell chromosome.

Inventive vectors V, V', etc. are typically prepared by inserting at least one inventive nucleic acid N, N', etc. into suitable vectors. Such suitable vectors are known to a skilled person and may be reviewed e.g. in "Cloning Vectors" (Eds. Pouwels P. H. et al. Elsevier, Amsterdam-New York-Oxford, 1985, ISBN 0 444 904018). Suitable vectors are also intended to include any vector known to a skilled person, such as plasmids, phages, viruses such as SV40, CMV, Baculo virus, Adeno virus, Sindbis virus, transposons, IS-elements, phasmids, phagemides, cosmides, linear or circular DNA. Such vectors can be replicated autonomously in a host organism or replicated chromosomally. For integration in mammalian cells linear DNA is typically used. Preferably, the vector type used for the present invention corresponds to the specific host cell requirements. Suitable commercially available expression vectors, into which the inventive nucleic acids N, N', etc. may be inserted, include pSPORT, pBluescriptIISK, the baculovirus expression vector pBlueBac, and the prokaryotic expression vector pcDNAII, all of which may be obtained from Invitrogen Corp., San Diego, CA.

An inventive vector V typically combines the inventive nucleic acid sequences N, N', etc., with other elements, which control expression of the encoded inventive fusion proteins A, A', etc.. Such additional elements are preferably selected from regulation sequences, origins of replication (if the vectors are replicated autonomously) and marker genes. Regulation sequences in the scope of the present invention are any elements known to a skilled person having an impact on expression on transcription and/or translation of inventive nucleic acids N, N', etc.. Regulation sequences include apart from promoter sequences so-called enhancer sequences, which may lead to an increased expression due to enhanced interaction between RNA polymerase and DNA. Further regulation sequences of inventive vectors V, V', etc. are transcriptional regulatory and translational initiation signals, so-called, "terminator sequences", "stability leader sequences", etc. or partial sequences thereof.

Generally, all naturally occuring promoters may be used in an expression vector V according to the present invention. Such promoters may be selected from any eukaryotic, prokaryotic, viral, bacterial, plant, human or animal, e.g. mammalian promoters. Suitable promoters include, for example, the cytomegalovirus promoter, the lacZ promoter, the gal 10 promoter and the AcMNPV polyhedral promoter, promoters such as cos-, tac-, trp-, tet-, trp-tet-, Ipp-, lac-, lpp-lac-, laclq-, T7-, T5-, T3-, gal-, trc-, ara-, SV40-, SP6, I-PR- or the I-PL-promoter, advantageously being found in gram-negative bacteria. Additionally, promoters may be obtained from gram-positive promoters such as amy and SPO2, yeast promoters, such as ADC1, MFa, AC, P-60, CYC1, GAPDH or mammalian promoters such as CaM-Kinasell, CMV, Nestin, L7, BDNF, NF, MBP, NSE, beta-globin, GFAP, GAP43, tyrosine hydroxylase, Kainat-receptor-subunit 1, glutamate-receptor-subunit B. Promoter elements as contained in an inventive vector V also may be obtained in association with native nucleic acid sequences for one of components (a), (b) and/or (c) of the inventive fusion protein, preferably component (a). Finally, synthetic promoters may be used advantageously.

Promoter sequences may also be inducible, to allow modulation of expression (e.g., by the presence or absence of nutrients or other inducers in the growth medium). One example is the lac operon obtained from bacteriophage lambda plac5, which can be induced by IPTG.

Finally, a promoter as defined above is preferably linked with an inventive nucleic acid N, N', etc., such that the promoter is positioned 5' "upstream" of the inventive nucleic acid N, N', etc..

Enhancer sequences for upregulating expression of inventive nucleic acids N, N', etc. are preferably another constituent of an inventive vector V, V', etc.. Such enhancer sequences are typically located in the non-coding 3' region of the vector. Enhancer sequences as employed herewith may be obtained from any eukaryotic, prokaryotic, viral, bacterial, plant, human or animal, e.g. mammalian hosts, preferably in association with the corresponding promoters as defined above.

Furthermore, an inventive vector V may contain transcriptional and/or translational signals, preferably transcriptional and/or translational signals recognized by an appropriate host, such as transcriptional regulatory and translational initiation signals. Transcriptional and/or translational signals may be obtained from any eukaryotic, prokaryotic, viral, bacterial, plant, human or animal, e.g. mammalian hosts, preferably in association with the corresponding promoters as defined above. A wide variety of transcriptional and translational regulatory sequences may be employed herein, depending upon the nature of the host. To the extent that the host cells recognizes the transcriptional regulatory and translational initiation signals associated with one of components (a), (b) and/or (c), the 5' region adjacent to the native coding nucleic acid sequence of any of components (a), (b) and/or (c) may be retained and employed for transcriptional and translational regulation in an inventive vector V. This region typically will include those sequences involved with initiation of transcription and translation, such as the TATA box, capping sequence, CAAT sequence, and the like. Typically, this region will be at least about 150 base pairs long, more typically about 200 bp, and rarely exceeding about 1 to 2 kb.

Transcriptional initiation regulatory signals may be selected that allow to control repression or activation such that expression of the genes can be modulated. One such controllable modulation technique is the use of regulatory signals that are temperature-sensitive in order to repress or initiate expressionby changing the temperature. Another controllable modulation technique is the use of regulatory signals that are sensitive to certain chemicals. Transcription and/or translational signals also include transcriptional termination regulatory sequences, such as a stop signal and a polyadenylated region. Preferably, transcriptional termination regulatory sequences are located in the non-coding 3' region of an inventive vector containing the nucleic acid sequence N, N' etc.. Suitable termination sequences include, for example, the bovine growth hormone, SV40, lacZ and AcMNPV polyhedral polyadenylation signals.

The inventive expression vectors may also include other sequences for optimal expression of the desired inventive fusion proteins. Such sequences include stability leader sequences, which provide for stability of the expression product; secretory leader sequences, which provide for secretion of the expression product; and restriction enzyme recognition sequences, which provide sites for cleavage by restriction endonucleases. All of these materials are known in the art and are commercially available (see, for example, Okayama (1983), Mol. Cell. Biol., 3: 280).

Additionally to regulation sequences as defined above, an autonomously replicating inventive vector typically comprises an origin of replication. Suitable origins of replication include, for example, ColE1, pSC101, SV40 and M13 origins of replication.

Finally, marker genes may also be contained in an inventive vector V. Such marker genes preferably allow phenotypic selection of transformed host cells. A marker gene may provide prototrophy to an auxotrophic host, biocide resistance (e.g. antibiotic resistance genes) and the like. The selectable marker gene may either be directly linked to the nucleic acid sequence N, N', etc. to be expressed, or may be introduced into the same cell by co-transfection. Examples of selectable markers include neomycin, ampicillin, hygromycin resistance and the like.

Another embodiment of the present invention refers to host cells C, being transformed with at least one inventive vector V. Advantageously, an inventive host cell which is used as an expression system in the present invention may be formed by combining a host cell as defined above and at least one inventive expression vector. A host cell C is preferably transformed with at least one inventive vector V, V', etc., having at least one nucleic acid sequence N, N', etc. encoding inventive fusion protein(s) A, A', etc.. An inventive host cell C is typically transformed with just one inventive vector V, if vector V contains at least two nucleic acids N and N', encoding fusion proteins A and A'. In an alternative embodiment, a host cell C is transformed with at least two different inventive vectors V and V'. In that case each vector V and V' typically contains just one inventive nucleic acid sequence N or N'.

As disclosed above, host cells of the invention are to be examined for the absence/presence of fluorescence signals resulting from the inventive fusion proteins. If a signal based on complementary components (c) and (c') of the fusion proteins A and A' is detected, that signal indicates an interaction between components (a) and (a') of both fusion proteins. In the absence of that fluorescence signal, components (a) and (a') of fusion proteins A or A', respectively, do not bind to one another. However, fluorescence of components (b) and (b') allows to separately localize each type of fusion protein in the cell.

Host cells are encompassed by the present invention as well, comprising three or more fusion proteins. E.g., if fusion proteins A, A' and A" are expressed in inventive host cells, these fusion proteins may comprise components (a), (a') and (a"), whereby both components (a') and (a") are capable of interacting with component (a). In such a situation a competitive binding of components (a') and (a") for component (a) occurs. Preferably, components (c') and (c") of fusion proteins A' and A" will be identical. Consequently, each of components (c') and (c") is capable to regain their fluorescence activity by binding to component (c) of fusion protein A. More preferably, fusion proteins A, A' and A" may comprise different components (b), (b') and (b"), allowing a person skilled in the art to localize any of fusion proteins A, A' and/or A" in the host cell.

Alternatively, the present inventive method may be used for the analysis of trimers, tetramers, etc.... multimers, in particular heterotrimers, heterotetramers, etc.. According to this embodiment, components (c), (c') and (c"), etc. are different portions of one fluorochrome protein as defined above and capable to regain their fluorescence activity upon assembly of all fusion proteins A, A', A", etc.. Thereby, the interaction of components (a), (a') and (a"), etc. of the inventive fusion proteins A, A', A", etc., when forming e.g. a heterotrimer, may be analysed.

Suitable host cells are to be understood herein as any cell, allowing expression of single DNA sequences according to the present invention or of DNA sequences in combination with additional sequences, particularly with regulation sequences. Any cultivated eukaryotic (yeast, mammalian cells), prokaryotic, viral, bacterial, plant, human and animal cell line may be used as a host cell, in particular cell lines of the immune system being impaired by infections, such as cell lines of the immune system impaired by HIV. Preferred host cells are bacteria, such as Eschericia coli., eukaryotic microorganisms, such as Saccharomyces cerevisiae (Stinchcomb et al., Nature, 282:39, (1997)).

In a preferred embodiment, cells from multi-cellular organisms are selected as host cells for expression of nucleic acid sequences according to the present invention. Cells from multi-cellular organisms are particularly preferred, if post-translational modifications, e.g. glycosylation of the encoded proteins, are required (N and/or O coupled). In contrast to prokaryotic cells, higher eukaryotic cells may permit these modifications to occur. The skilled person is aware of a plurality of established cell lines suitable for this purpose, e.g. 293T (embryonic kidney cell line), HeLa (human cervix carcinoma cells) and further cell lines, in particular cell lines established for laboratory use, such as HEK293-, Sf9- or COS-cells. Particularly preferred are human cells, more preferably cells of the immune system or adult stem cells, such as stem cells of the hematopoietic system (derived from bone marrow).

Another embodiment of the present invention refers to a method for detecting protein-protein-interactions. The method according to the present invention preferably comprises the following steps: I) providing at least one vector V according to the present invention, either II1) by transfection of a host cell with one vector type V provided by step (I), wherein this vector contains at least two inventive nucleic acids N and N', or II2) by transfection of a host cell with at least two different vectors types V, V' etc., wherein each vector comprises an inventive nucleic acid sequence N, N' etc.. As disclosed above, the inventive nucleic acids N and N' encode fusion proteins A and A'. The fluorochrome groups (components (b) and (b')) contained in these inventive fusion proteins A and A' are preferably not identical, in order to allow to localize independently each fusion protein A or A', etc. in the cell. As disclosed above, each single fusion protein A or A', etc. contains in its component (c) just a portion of a fluorochrome protein. Upon assembly of fusion proteins A and A', which is dependent on the interaction of their components (a) and (a'), the full-length fluorochrome protein composed of two separated portions (c) and (c') may emit a fluorescence signal, which is observed in step (III) of the inventive method. Therefore, if an interaction occurs between fusion proteins A and A' via their components (a) and (a'), three fluorescent signals can be observed. One signal results from fluorochrome protein portions (c) and (c') regaining their fluorescent properties, and two separate fluorescence signals result from components (b) and (b'), respectively, contained in the inventive fusion proteins A and A'.

In contrast, if both fusion proteins A and A' have successfully been expressed, but no interaction occurs between fusion proteins A and A' via their components (a) and (a'), no regain of the fluorescence of fluorochrome protein portions (c) and (c') occurs. Step (III) will allow to detect two separate fluorescence signals resulting from components (b) and (b'), respectively. Just one fluorescence signal will appear, if just one fusion protein is expressed in the host cell, e.g. because the other fusion protein is - for whatever reason - not expressed.

Detection in step (III) of the inventive method may be carried out by any method being suitable for detecting fluorescence signals. Such methods include distinct generation of fluorescence signals, e.g. by activating fluorochrome groups, and detecting generated fluorescence signals subsequently. Simultaneous or time-staggered generation and detection of fluorescence signals of fluorochrome groups is within the scope of this invention as well. It is preferred to carry out detection step (III) with a laser-induced fluorescence detection (LIF), a laser-induced time-staggered fluorescence detection (LI2F), a Fluorescence Lifetime Imaging Microscopy (FLIM), a spectrophotometry, flow cytometry, or a white fluid fluorescence spectroscopy. Preferably, living host cells transfected accordingly may be depicted by step (III) three-dimensionally in a micrometer solution and analysed thereafter.

When generating a fluorescence signal by any of the aforementioned fluorescence detection methods, preferably the excitation wavelength is selected such as to specifically excite (potentially) regained fluorescence activity of components (c) and (c'). Excitation may evoke the emission of a fluorescence signal (signal 1) from these components. If no fluorescence signal 1 is observed the excitation wavelength is typically shifted to excite a fluorescence signal from components (b) (signal 2) and/or components (b') (signal 3). However, signals 2 and/or 3 may be obtained prior to excitation of signal 1. Alternatively, the excitation of all fluorochromes may be carried out simultaneously.

Another object of the present invention refers to a method directed to screening for modulators, i.e. inhibitors or enhancers, of protein-protein interactions. The inventive method may be carried out *in vivo,* i.e. in living cells, as well as *in vitro*, e.g. in cell lysates or in cell free assays. If carried out in vivo, the inventive method for screening modulators preferably comprises the following steps: (I) providing at least one inventive host cell type C containing and expressing a first inventive fusion protein A and a second inventive fusion protein A', (II) providing and adding at least one test compound to these host cells, and (III) detecting the altered fluorescence signal of the fusion proteins A and A' expressed in the cells. Preferably fusion proteins A and A' are fusion proteins known to interact with each other.

Host cells C as provided by step (I) have typically been transfected with at least one inventive vector type V as defined above. If an inventive host cell has been transfected with just one inventive vector type V, the vector typically encodes at least two inventive fusion proteins A and A'. If at least two vectors V and V' have been transfected into host cells type C, each vector V and V' preferably encodes just one fusion protein, e.g. two fusion proteins A and A'. In both alternatives preferably at least two fusion proteins A and A' are expressed by the inventive host cell. Typically, each fusion protein contains a component (a) or (a'), etc., respectively, representing proteins being engaged in protein-protein-interactions. Components (a) and (a') of fusion proteins A and A' used for the inventive screening method are preferably capable to interact with each other as discussed above. If components (a) and (a') of the inventive fusion proteins A and A' interact with each other, three fluorescence signals are observed, provided that interaction of components (a) and (a') is not disturbed. As explained above, one signal results from fluorochrome protein portions (c) and (c'), and two other fluorescence signals result from components (b) and (b') each.

A test compound as provided in step (II) of the above inventive method is preferably any compound, which may presumably enhance or inhibit the interaction of components (a) and (a') of the inventive fusion proteins A and A'. Preferably, the test compound may inhibit the interaction of components (a) and (a') of the inventive fusion proteins A and A', e.g. by binding to the binding site of components (a) and/or (a') or by inducing a conformational change of at least one of components (a) or (a') of fusion proteins A or A'. Alternatively, the test compound may enhance the interaction of components (a) and (a') of the inventive fusion proteins A and A', e.g. by stabilizing the complex formed by components (a) and (a') of fusion proteins A and A'.

Test compounds as used in the inventive method may be provided from any known compound library, preferably from small molecule compound libraries, containing inorganic and organic compounds, peptides, proteins, hormones, antibodies, etc.. Alternatively, test compounds may be derived from any biological source, such as plants, tissues, body fluids, such as blood, lymph, etc. If the modulatory potential of test compounds from biological sources is analysed, these sources are preferably homogenized prior to addition to the cells. Thereby, the test compound is added to the cells in a defined and reproducible manner. Such homogenized sources are typically cell suspensions and may contain cells, cell fragments, etc.. If the homogenized material is not to be added as such, test compounds may be isolated or extracted from these homogenized sources preferably prior to (or eventually subsequent to) addition of the cells in step (I) by conventional biochemical methods, such as chromatography, e.g. affinity chromatography (HPLC, FPLC, ...), size exclusion chromatography, etc., as well as by cell sorting assays, antibody detection, etc..

Typically, just one test compound species is added in step (II) of the inventive screening method (e.g. if test compounds derived from a compound library, or isolated compounds derived from any biological source). However, more than one test compound may be added in step (II), e.g. 2-10, 2-50, 2-100 or more test compound species added to the sample. This embodiment allows several test compound species to be screened simultaneously.

In final step (III), detection of the (altered) fluorescence signal(s) of the at least two fusion proteins A and A' in the host cell is carried out, preferably by any of the afore mentioned methods for detecting fluorescence. Preferably, the detection method of step (III) is directed to record the fluorescence signal emitted by the fluorochrome protein components (c) and (c'). Moreover, a shift of fluorescence signal intensity by components (c) and (c') is observed, when compared to fluorescence measurement without addition of test compound. As mentioned above, three fluorescence signals can be observed, if components (a) and (a') of the inventive fusion proteins A and A' interact with each other and interaction of components (a) and (a') is not impaired by the test compound. In summary, one signal (signal 1) results from fluorochrome protein portions (c) and (c'), while two fluorescence signals (signals 2 and 3) are due to the fluorescence of components (b) and (b')). In contrast, if interaction of components (a) and (a') is impaired by a test compound as provided in step (II), a change of fluorescence signal 1 is to be expected due to impaired assembly of fluorochrome protein portions (c) and (c'). Intensity of signals 2 and 3 resulting from components (b) and (b') typically remain unchanged, since these signals are independent upon fusion protein interactions. If e.g. the test compound, as added to the cells in step (II), is an inhibitor of the interaction of components (a) and (a') of fusion proteins A and A', a decrease in signal intensity of signal 1 will be observed. That decrease is due to (partial) interaction loss of protein portions (c) and (c'). Hence, decrease of signal 1 intensity may typically range from slight signal intensity loss to signal extinction. Signal extinction is to be expected, if no interaction occurs between components (a) and (a') of fusion proteins A and A'. If a total extinction of signal 1 occurs, just two fluorescence signals of components (b) and (b') (signals 2 and 3) remain. Complete extinction of fluorescence signal 1 is due to inhibitory effects of the test compound disrupting interactions of components (a) and (a').

If the test compound is an enhancer of the interaction of components (a) and (a'), signal 1 internsity increases, when compared to fluorescence measurement without addition of test compound. Consequently, three fluorescence signals are observed, if components (a) and (a') of fusion proteins A and A' interact with each other, whereby signal 1 of components (c) and (c') is enhanced as compared to signal 1 of components in absence of the test compound.

In an optional step (IV) of the inventive screening method, the test compound as used in the present inventive method, may be isolated. Step (IV) may turn out to be of major importance, if the test compound is not added to the cells in step (II) in its isolated form, e.g. if the chemical nature of the test compound was not determined before addition. E.g. the homogenized raw material (initially added in step (II)) is fractionated. Each single fraction is then tested for its activity by the inventive screening methods. The active fraction(s) may be subjected to further rounds of fractionating and testing as disclosed above.

The same isolation procedure may be applied, if more than one test compound, obtainable e.g. from compound libraries, is added in step (II) to one sample. Then, e.g. as with the raw material, the solution of step (II), containing various test compounds, is fractionated. Each single fraction is then tested for its activity by the inventive screening method. The active fraction(s) may be subjected to further rounds of fractionating and testing as disclosed above.

Alternatively or additionally, the isolated test compound(s) may be identified as a complex with its(their) associated inventive fusion protein(s), e.g. after or instead of fractionating as defined by step (IV). Therefore, conventional biochemical methods may be applied, such as chromatography, e.g. affinity chromatography (HPLC, FPLC, ...), size exclusion chromatography, gas chromatography, sorting assays, antibody detection, as well as biophysical methods such as infrared spectrometry, NMR, X-ray analysis, etc..

In alternative of the inventive screening method, the method may be carried out *in vitro*, e.g. in cell lysates or in cell free assays. If carried out *in vitro,* the inventive method for screening modulators preferably comprises the following steps: (I) providing at least one inventive fusion protein A and a second inventive fusion protein A', (II) providing and adding at least one test compound to e.g. cell free systems, and (III) detecting the altered fluorescence signal of the fusion proteins A and A' expressed in cell free systems. Inventive fusion protein A and A' and test compounds as used in steps (I) and (II) are preferably as defined above. More preferably, inventive fusion protein A and A' and test compounds may be added in any order, e.g. starting with inventive fusion protein A, then adding fusion protein A' and finally adding test compound(s). Alternatively, test compound(s) may be added first and afterwards fusion proteins A and A' are be added. Simultaneous addition of test compound(s) and/or fusion proteins A and A' is also contemplated. Furthermore, step (III) of the inventive screening method may be carried out as outlined above for step (III) of the analogous in vivo procedure.

Preferably, the inventive in vitro screening method is carried out by using affinity assays, such as (column) chromatography, ELISA, etc.. Therefore, at least one inventive fusion protein A and/or A' is labelled with a group for immobilisation on a solid phase as defined above. E.g. fusion protein A may then be bound to a solid phase. A solid phase is meant to be any surface, to which a fusion protein may be immobilised via a label or via its affinity. A solid phase is meant to be nylon wool, beads, microbeads, sepharose, sephadex, etc.. After immobilization of at least one fusion protein (e.g. fusion protein A), either test compound(s) or fusion protein A' is (are) added. After forming a complex between at least one fusion protein and at least one test compound. Test compound action may be determined either by inhibition of complex formation (inhibitory action of test compounds) or by enhancement of complex formation. In order to detect active test compounds, complexes of fusion proteins A/A' or uncomplexed fusion protein A may be eluted from the e.g. column. The complexes or uncomplexed immobilized fusion proteins A/A' may be eluted from the solid phase, preferably after a washing step. The fractions obtained after elution may the be isolated and/or identified as disclosed above for inventive in vivo screening assays.

Another subject of the present invention is a method for determining interactions of a first (known) protein with a second (unknown) protein using a library of cells. The inventive method preferably comprises the following steps of: (I) generating a host cell library LC, whereby each cell comprises (l1) an inventive fusion protein A; and (12) an inventive fusion protein A'; (II) screening the host cell library for host cells by detecting fluorescence signal(s) of the expressed fusion proteins A and A'.

In step (I) of the inventive screening method a host cell library LC generated. Each host cell comprises a (known) fusion protein type A (which is identical in each host cell). Moreover, the library host cells to be used comprises a second (unknown) fusion protein A' which is different in each library host cell. A host cell library LC as used in the inventive screening method is preferably a library comprising expression constructs prepared from randomly assembled expressible inventive nucleic acid sequences N, N', etc.. These inventive nucleic acid sequences may be derived from a plurality of species of donor organisms. Typically, they are operably associated with regulatory regions of at least one vector V, V', etc. that controls expression of the expressible nucleic acid sequences N, N', etc.. The inventive library LC is preferably prepared by providing at least one expressible nucleic acid sequence N, N', etc. as defined above. These nucleic acid sequences are typically inserted into inventive vectors V, V', etc.. Finally, the inventive vectors V, V', etc. are preferably transfected into host cells C, C', etc.. A library as used in the inventive screening method typically comprises at least 20 individual host cells C, C', etc., more preferably at least 100, 1.000 or 10.000 individual host cells C, C', etc.. Most preferably, such a library comprises at least 100.000, 1.000.000 or 1.000.000.000 host cells C, C', etc..

A host cell library LC as generated in step (I) of the inventive screening method may be amplified, replicated, and stored. Amplification is preferably carried out by introducing entry vectors containing expressible nucleic acid sequences N, N', etc. in an initial host cell C such as to allow to produce multiple clones of the expressible nucleic acid sequences N, N', etc.. Replication refers to picking and growing of individual clones in the library. An inventive library LC may be stored and retrieved by any techniques available in the art that is appropriate for the host cell C. Thus, inventive libraries LC are an effective means of capturing and preserving the genetic resources of donor organisms, which may be accessed repeatedly in a drug discovery program or other discovery programs.

The host cell library LC as generated in step (I) may be a random combination of promoter and expressible nucleic acid sequences N, N', etc. made from a two dimensional array of promoters and expressible nucleic acid sequences N, N', etc.. Thereby, it is possible to get-in principle-all expressible nucleic acid sequences N, N', etc. from a given pool represented in a library under the control of different promoters.

Generating host cell libraries LC in step (I) may be carried out by transfecting vectors V into inventive host cells C. Transfection methods are known in the art (see e.g. Maniatis et al., 2001, supra) and include spontaneous uptake methods, conventional chemical methods, such as the polyamidoamine dendrimer method, the DEAE-dextran method or the calcium phosphate method, physical methods such as microinjection, electroporation, biolistic particle delivery, etc.. The selection of host cells used for library construction will typically depend on the nature of the proteins to be tested for their interaction partners. E.g., proteins to be tested for their interaction partners in living cells will typically require appropriate cells as a basis for library construction. In other words, host cells C are typically selected such as to provide the naturally occurring environment for components (a) and (a') of fusion proteins A and A'.

If an inventive host cell is transfected with just one inventive vector type V, the vector typically encodes a first fusion protein A and a second fusion protein A'. If at least two vectors V and V' have been transfected into host cells C and C', etc., each vector V, V', etc. typically encodes fusion protein A or fusion protein A', respectively.

Component (a) of the first fusion protein A as provided with the host cells C in step (I) is preferably any human protein or protein fragment the interaction of which shall be analysed. E.g., component (a) may any prokaryotic or eukaryotic protein, preferably viral, bacterial, plant, human or animal protein. Component (a) of the first fusion protein A may be present in its full length form or as a fragment of the full-length sequence, e.g. domains. "Known protein" is meant to be any protein, which is known in the art and/or which has already been isolated in its full length form or in partial sequences, e.g. domains. As mentioned above, a known protein, e.g. component (a) of the first fusion protein A, is preferably identical in each host cell of the host cell library LC.

Component (a') of the second fusion protein A' as provided with the host cells C in step (I) is an unknown protein, the interaction of which with a known protein A is to be tested. "Unknown protein" is meant to be any protein, which is not yet characterized with respect to its interaction properties in the art and/or which has not been isolated in its full length form or as partial sequence, e.g. domains. Component (a) may be derived from any prokaryotic or eukaryotic protein, preferably viral, bacterial, plant, human or animal proteins. As with component (a), component (a') of fusion protein A' may be present in its full length form or as a fragment of the full-length sequence, e.g. domains. This is to say that components (a') of this second fusion proteins A' typically may be any protein of interest, any protein or peptide sequence as contained in protein or peptide libraries or databases, any translated protein sequence as encoded by sequences contained in nucleic acid libraries or databases, e.g. for genomic DNA, subgenomic DNA, cDNA, synthetic DNA sequences, etc. and combinations thereof. Component (a') of the second fusion protein A' may be obtained by isolation from biological samples, such as tissues or body liquids as defined above, e.g. blood, lymph, etc.. Preferably, the reading frame of the nucleotide sequences encoding component (a') of the second fusion protein A' is not interrupted by a stop codon.

In step (II) of the inventive method the library is screened for host cells exhibiting fluorescence signal regain of components (c) and (c') of fusion protein A and/or A', indicating an interaction of components (a) and/or (a'). Detection of fluorescence signal(s) of the first fusion protein A and the second fusion protein A' in the host cell is carried out, preferably by any of the afore mentioned methods for detecting fluorescence.

In optional step (III), host cells are isolated which were identified as hits by fluorescence due to components (c) and (c'). Any selection method as known in art may be used, including conventional cell sorting assays, antibody binding assays, affinity assays such as affinity chromatography assays, etc. to isolate the cells and, therefore, to identify components (a') of the (unknown) fusion protein A' as interacting partner.

The present invention refers to vector libraries LV and to host cell libraries LC preferably containing and expressing inventive fusion proteins A, A', etc..

Another embodiment of the present invention refers to a kit for screening modulators, i.e. inhibitors or enhancers, of protein-protein interactions. Such a kit preferably comprises inventive vector(s) of general type V and V' or inventive host cell(s) of general type C as defined above. More preferably, the inventive kit for screening modulators of protein-protein interactions comprises as constituent (1) inventive vector(s) V, wherein vector(s) V encodes at least two inventive fusion proteins A and A', or alternatively at least two inventive vectors V and V' as constituent (1'), wherein each vector encodes just one inventive fusion protein A or A'. Further, the inventive kit optionally comprises as constituent (2) instructions for use.

Finally, the invention refers to use of inventive fusion proteins A, inventive nucleic acids N, inventive vectors V, inventive host cells C, inventive kits, inventive host cell libraries LC. Such uses encompass detection of protein-protein-interactions as well as screening modulators, i.e. inhibitors or enhancers of protein-protein interactions. Detection of interactions of a first protein with a second protein are also encompassed. Preferably, the inventive fusion proteins A, nucleic acids N, vectors V and/or kits may be used in living cells and in cell lysates as well as in other cell-free environments, buffers, etc. Further, they may be used in addition to any state-of-the-art determination of protein-protein-interactions in cells or cell lysates, e.g. conventional methods, automatized methods such as High-Throughput-Screening methods (HTS), etc.

### Description of the Figures:

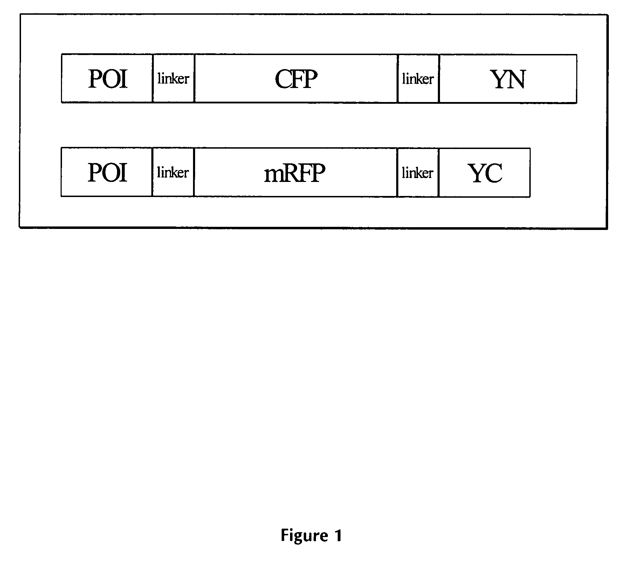

- Figure 1:: Diagram of the exBiFC principle. The fusion protein in the upper field displays component (a) (POI = protein of interest/protein to be tested), being fused to CFP via a linker, wherein CFP is fused via a linker with the N-terminus of YFP. The fusion protein in the lower field illustrates the POI fused with mRFP via a linker, wherein mRFP is fused via a linker with the N-terminus of YFP.

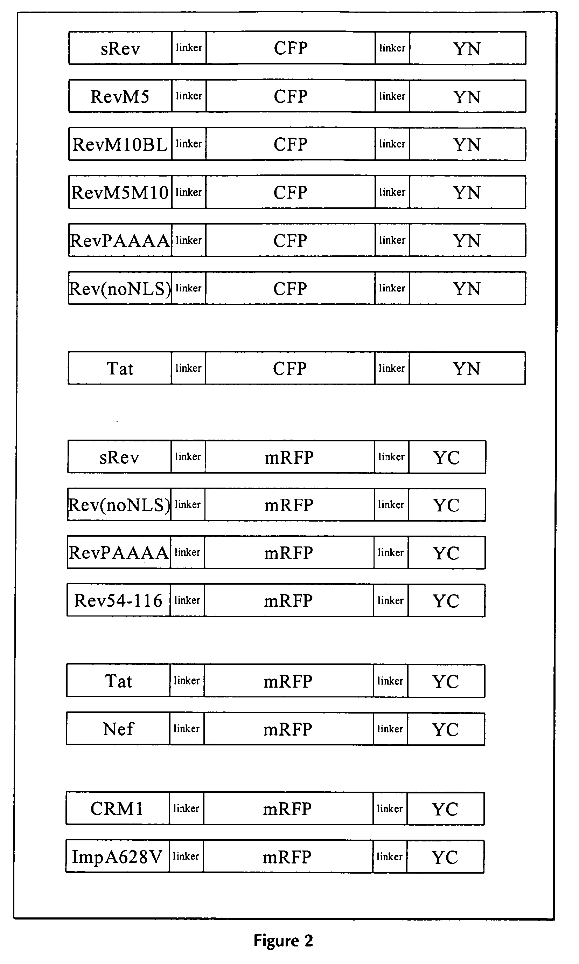

- Figure 2:: Overview of the cloned constructs of all exemplary exBiFC final plasmids (not scaled).

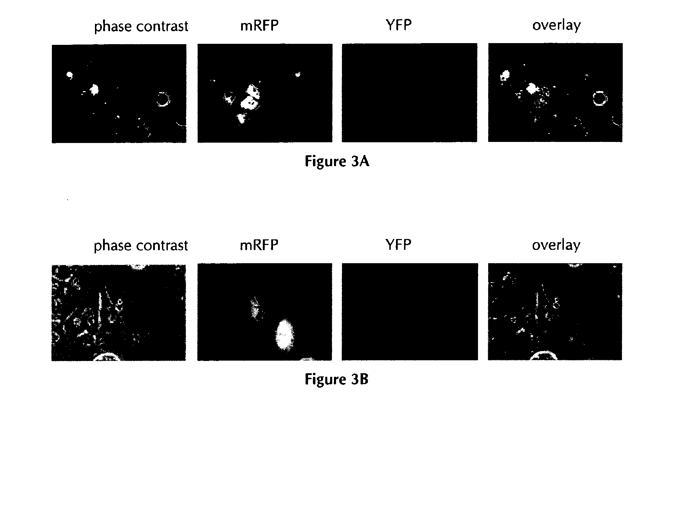

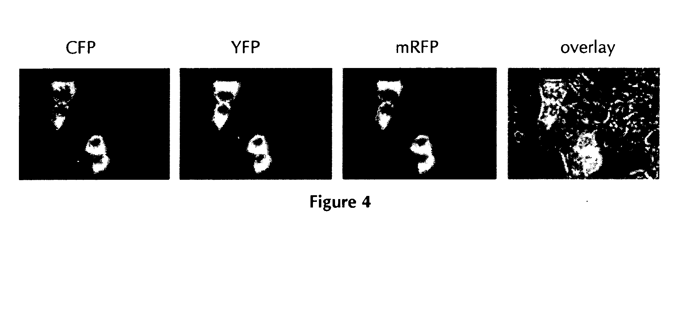

- Figure 3A:: Fluorescence microscope images from HeLa cells, transfected with pCMV-CFP-YN. The first image depicts the phase contrast, the second the fluorescence intensity in the CFP-channel, the third image shows the fluorescence intensity in the YFP channel. The last image shows an overlay image of the fluorescence of all channels by using a phase contrast image. Any of the depicted images in Figure 3A are images prepared with the epifluorescence microscope (Cellobserver).

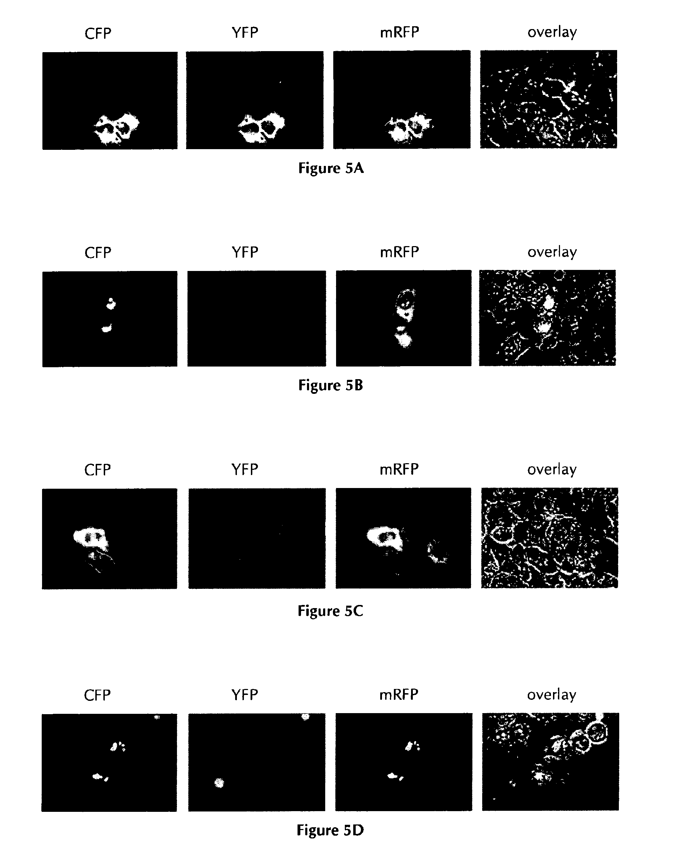

- Figure 3B:: Fluorescence microscope images from HeLa cells, transfected with pCMV-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 4:: Fluorescence microscope images from HeLa cells, cotransfected with pCMV-sRev-CFP-YN and pCMV-sRev-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5A:: Fluorescence miscroscope images from HeLa cells, cotransfected with pCMV-sRev-CFP-YN and pFED-Rev54-116-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5B:: Fluorescence microscope images from HeLa cells, cotransfected with pCMV-RevM10BL-CFP-YN and pCMV-sRev-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5C:: Fluorescence microscope images from HeLa cells, cotransfected with pCMV-sRev-CFP-YN and pCMV-Rev54-116-mRFP-Yc. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5D:: Fluorescence microscope images from HeLa cells, cotransfected with pCMV-sRev-CFP-YN and pCMV-RevPAAAA-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.

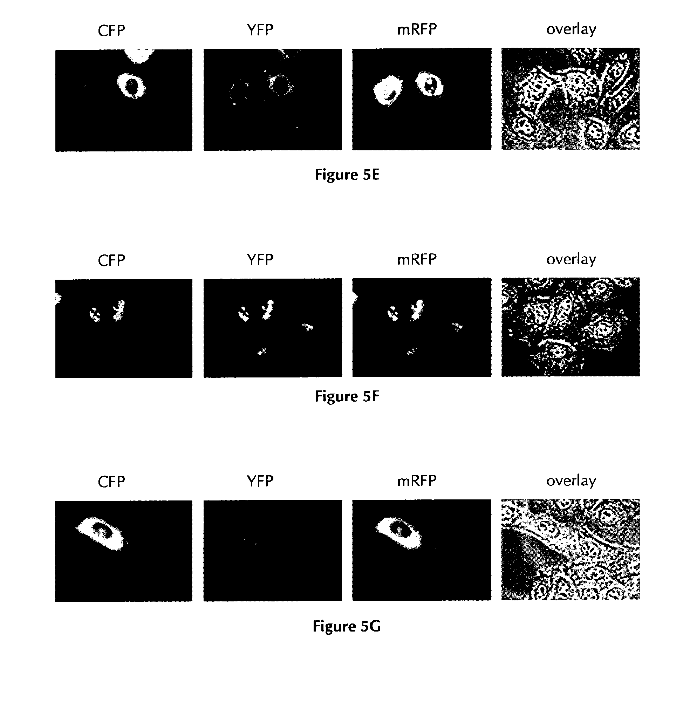

- Figure 5E:: Fluorescence microscope images from HeLa cells, cotransfected with pCMV-RevM5-CFP-YN and pCMV-RevPAAAA-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5F:: Fluorescence microscope images from HeLa cells, cotransfected with pCMV-RevM10BL-CFP-YN and pCMV-RevPAAAA-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 5G:: Fluorescence microscope images from HeLa cells, cotransfected with pCMV-RevM5-CFP-YN and pCMV-Rev54-116-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.

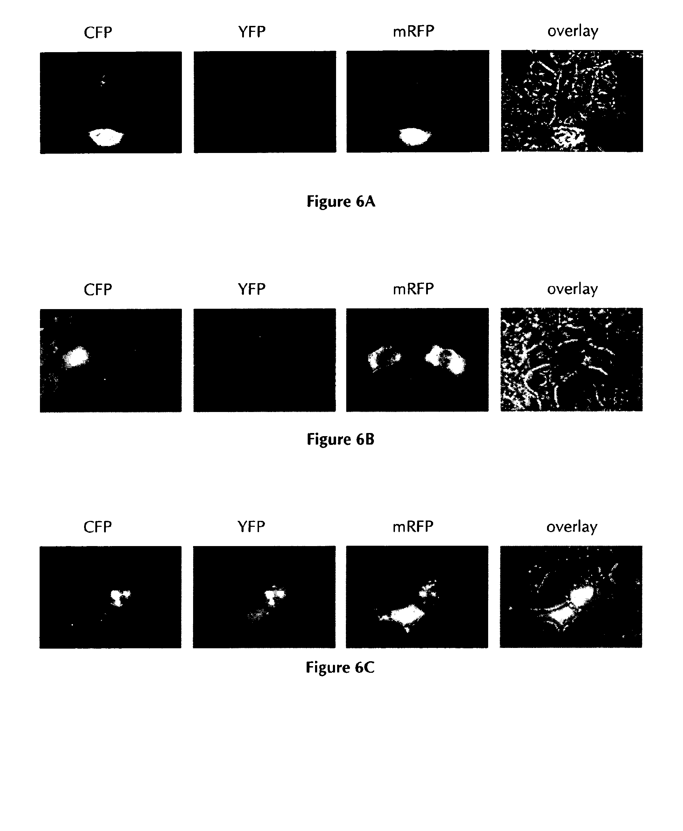

- Figure 6A:: Fluorescence microscope images from HeLa cells, cotransfected with pCMV-sRev-CFP-YN and pCMV-Nef-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 6B:: Fluorescence microscope images from HeLa cells, cotransfected with pCMV-Tat-CFP-YN and pCMV-sRev-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 6C:: Fluorescence microscope images from HeLa cells, cotransfected with pCMV-Tat-CFP-YN and pCMV-Nef-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.

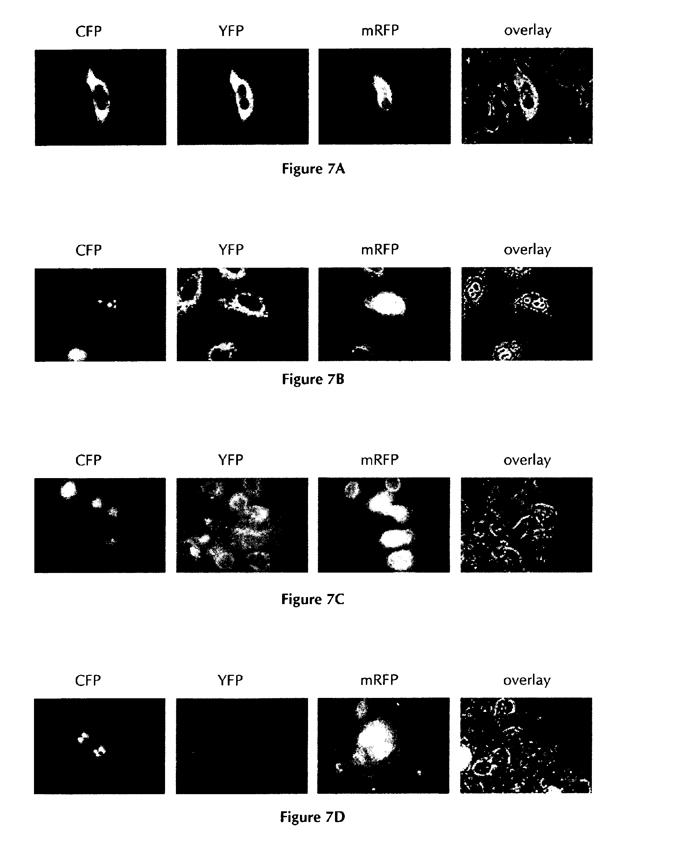

- Figure 7A:: Fluorescence microscope images from HeLa cells, cotransfected with pCMV-sRev-CFP-YN and pCMV-CRM1-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 7B:: Fluorescence microscope images from HeLa cells, cotransfected with pCMV-RevM10BL-CFP-YN and pCMV-CRM1-mRFP-YC. The first image depicts the phase contrast, the second the fluorescence intensity in the CFP-channel, the third the fluorescence intensity in the YFP channel. The last image shows an overlay image of the fluorescence of all channels by using a phase contrast image.
- Figure 7C:: Fluorescence microscope images from HeLa cells, cotransfected with pCMV-RevM5M10BL-CFP-YN and pCMV-CRM1-mRFP-YC. The first picture depicts the phase contrast, the second the fluorescence intensity in the CFP-channel, the third the fluorescence intensity in the YFP channel. The last picture shows an overlay image of the fluorescences of all channels with the phase contrast image.

- Figure 7D:: Fluorescence microscope images from HeLa cells, cotransfected with pCMV-RevPAAAA-CFP-YN and pCMV-CRM1-mRFP-YC. The first picture depicts the phase contrast, the second the fluorescence intensity in the CFP-channel, the third the fluorescence intensity in the YFP channel. The last picture shows an overlay image of the fluorescences of all channels with the phase contrast image.

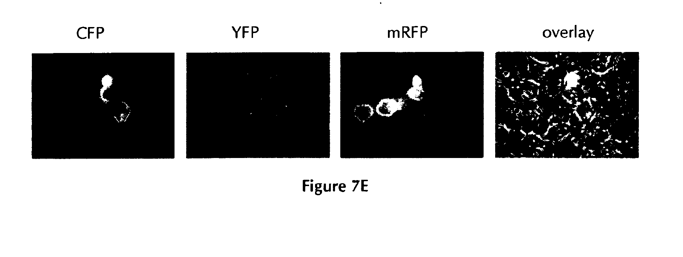

- Figure 7E:: Fluorescence microscope images from HeLa cells, cotransfected with pCMV-Rev(noNLS)-CFP-YN and pCMV-CRM1-mRFP-YC. The first picture depicts the phase contrast, the second the fluorescence intensity in the CFP-channel, the third the fluorescence intensity in the YFP channel. The last picture shows an overlay image of the fluorescences of all channels with the phase contrast image.

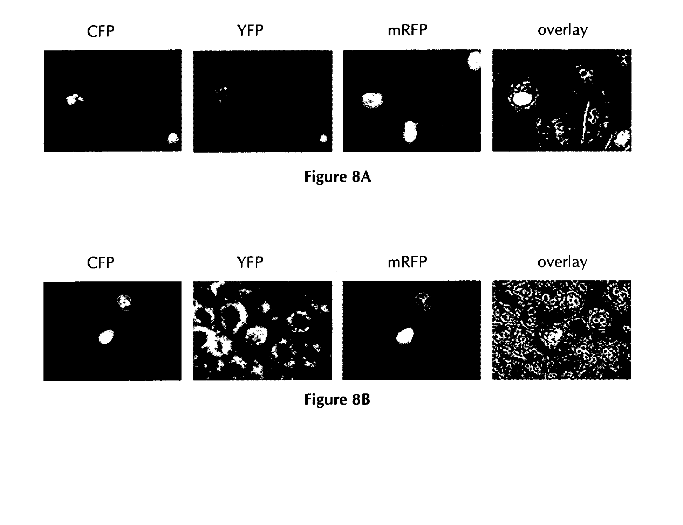

- Figure 8A:: Fluorescence microscope images from HeLa cells, cotransfected with pCMV-sRev-CFP-YN and pCMV-Importinβ(A628V)-mRFP-YC. The first picture depicts the phase contrast, the second the fluorescence intensity in the CFP-channel, the third the fluorescence intensity in the YFP channel. The last picture shows an overlay image of the fluorescences of all channels with the phase contrast image.
- Figure 8B:: Fluorescence microscope images from HeLa cells, cotransfected with pCMV-Tat-CFP-YN and pCMV-Importinβ(A628V)-mRFP-YC. The first picture depicts the phase contrast, the second the fluorescence intensity in the CFP-channel, the third the fluorescence intensity in the YFP channel. The last picture shows an overlay image of the fluorescences of all channels with the phase contrast image.

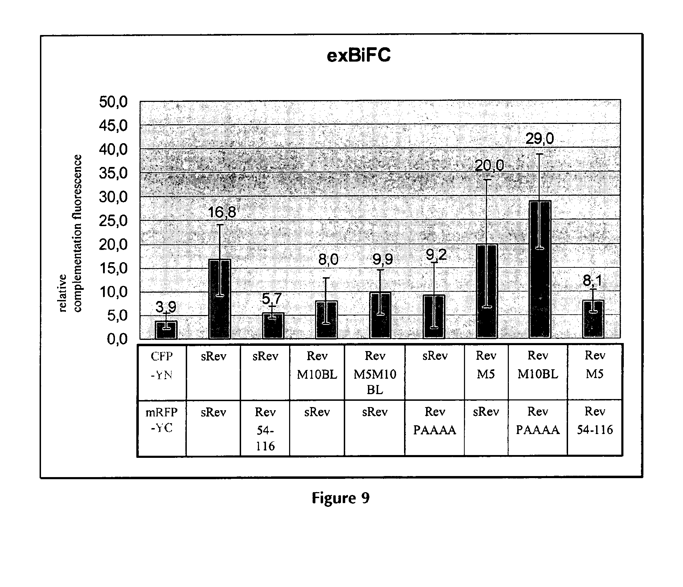

- Figure 9:: Bar plot of the analysis of cotransfection experiments with exBiFC plasmids from sRev and Ref-mutants. The legend of the bars indicates the transfected plasmids in abbreviated form. The first bar e.g. displays the result of the cotransfection of pCMV-CFP-YN and pCMV-mRFP-YC. The second bar displays the result of the cotransfection of pCMV-sRev-CFP-YN and pCMV-sRev-mRFP-YC, etc.. The first indicated protein is always related to -CFP-YN and the second indicated protein always refers to -mRFP-YC. The first transfection with CFP-YN and mRFP-YC individually displays the value for the background fluorescence of the exBiFC system, which is also indicated for all following analysis as an orientation. The second transfection with sRev-CFP-YN and sRev-mRFP-YC displays the positive control for all following transfection experiments, since it is known that sRev-molecules multimerise with each other. On the basis of these first results the interaction of the Proteins of lnterest(POls = proteins to be tested) of the following transfections are calculated. Accordingly the following transfections with the Proteins of Interest (POls)/proteins to be tested sRev + Rev54-116, RevM10 + sRev, RevM5M10 + sRev, as well as sRev + RevPAAAA display nearly or no interaction respectively. However, RevM5 + sRev as well as RevM10 + RevPAAAA apparently display an interaction, whereas RevM5 with Rev54-116 does not show any interaction.

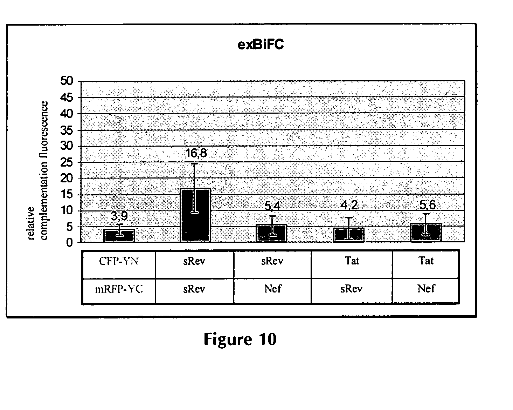

- Figure 10:: Bar plot of the analysis of cotransfection experiments with exBiFC plasmids from sRev, Tat and Nef. Fig. 10 discloses the analysis of transfection experiments with Rev, Tat and Nef. Furthermore, the results of the background fluorescence of the system (CFP-YN and mRFP-YC), as well as the positive control with sRev are displayed. On the basis of the results for the cotransfection experiments of sRev-CFP-YN+Nef-mRFP-YC, Tat-CFP-YN+sRev-mRFP-YC, as well as of Tat-CFP-YN+Nef-mRFP-YC, it is to be noted clearly that neither sRev interacts with Nef, nor Tat with sRev or Tat with Nef.

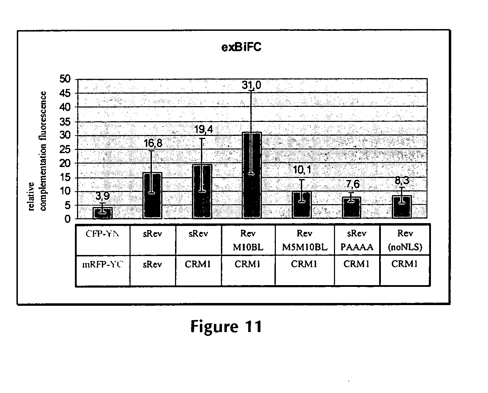

- Figure 11:: Bar plot of the analysis of cotransfection experiments with exBiFC plasmids from Rev and CRM1. Fig. 11 shows the results of the transfection from sRev and different Rev-mutants with the cellular export factor CRM1. Firstly, the background fluorescence and subsequently the positive control of the exBiFC system are shown. The next two cotransfection experiments from sRev as well as from RevM10 with the cellular export factor CRM1 display a significant interaction of the respective proteins on the basis of the values. The following three transfections from RevM5M10, RevPAAAA and Rev(noNLS) with CRM1 show no interaction of the three proteins with CRM1.

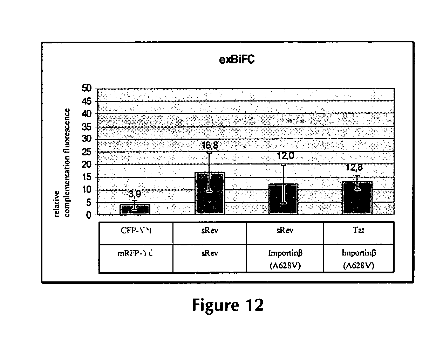

- Figure 12:: Bar plot of the analysis of cotransfection experiments with exBiFC plasmids from Rev, Tat and Importinβ(A628V). Fig. 12 displays the analysis of transfection experiments with Rev and Tat with the mutants of the cellular import protein Importinβ. Initially, the background fluorescence and the positive control of the exBiFC system are shown likewise. The results indicate a quite weak interaction of sRev and Tat with Importinβ(A628V).

### Examples:

The following non-limiting examples illustrate the present invention and do not limit the invention in any way. The examples provide the skilled person with a guidance for using the compounds and methods of the invention.

### 1. Microbiological Methods

### 1.1 Cultivation of human cell lines

All cell lines were cultivated at 37°C, 5% CO₂, under air saturated with humidity, in cell culture flasks (Nunc Solo Flask 185 cm²) with 10 ml medium (DMEM (Dolbecco's Modified Eagle Medium) with 10% FCS and 1% antibiotic/antimycotic). The medium further contained the indicator phenolic red, signalising the lowered pH value of the medium due to metabolised by turning yellow. Once the medium turned yellow or the cells were grown confluently to 80-90% the cells were passaged. Therfore, medium was taken off and the cells were washed with 5 ml PBS and about 1 ml Trypsin/EDTA was added. The cells detached from the bottom of the culture plate within 5 minutes at 37°C. The process was stopped by adding 9 ml medium, the cells were resuspended in medium and transferred 1:20 (HeLa cells, for purposes of the patent application obtained from ATCC (CRL-7923)) and 1:5 (U138MG-cells), respectively, into fresh medium. It was paid attention not to exceed a passage number of 30, since aging effects could occur with high passage numbers, which have a negative impact on experiments and measurement data. Frequently cells with lower passage numbers were defrosted for experiments in order to assure comparable conditions.

For transfection experiments the cells were dissolved as mentioned above from the bottom of the culture flask and resuspended in medium in order to count the cells in a counter box (Madaus Diagnostik). Therefor, one drop of the cell suspension was added to the counter box and the cells of a large square (= 16 small squares) were counted. This number multiplied with 10⁴ resulted in the cell number per ml for the initial suspension. Then a cell number from 1x10⁵ was seeded in a 35 mm plate with a glass bottom and cultivated in 2 ml medium.

### 1.2 Detection of fluorescent proteins in human cell lines

Fluorescent proteins in human cell lines were detected by using the inverse microscope Axiovert 200M (Cell Observer). In the following table different fluorescent proteins with the maximum of their absorption and emission wavelength are listed exemplary:

| Fluorochrome | Maximum of excitation wavelength | Maximum of emission wavelength |
|---|---|---|
| Cyan fluorescent protein (ECFP) | 434 | 477 |
| Yellow fluorescent protein (EYFP) | 514 | 527 |
| MRFP1 (monomeric Red Fluorescent Protein) | 584 | 607 |

The three fluorochrome ECFP, EYFP and mRFP1 (monomeric Red Fluorescent Protein) were exemplarily used in this work. After the transfection of the cells with the corresponding expression plasmid the cells usually were photographed after 48 h with the Cellobserver. Therefor, the camera was automatically controlled through the software and produced multi-channel images. The exposure time was adapted to the intensity of expression of the proteins coupled to the fluorochrome and to the transfected amount of DNA. In cases of weakly expressed proteins an aperture was additionally opened, assuring a fluorescence intensity of approximately 25% of the lamp output, in order to concentrate more light on the sample. The images were compressed into TIF-format. The further analysis was performed with the programme IPLab. The intensity of fluorescence signals were thereby adjusted to the background signal and analysed.

Cells showing a fluorescence signal in the CFP as well as in the mRFP channel were initially selected for analysis in the programme IPLab and borded manually with green colour. Subsequently an area in the cell-free region was determined and marked red in order to determine the background fluorescence of the single channels. Afterwards the fluorescence intensity of the single channels was measured in the respective channel including the corresponding background fluorescence. For each area the following three values were obtained: 1) Sum: Sum of the fluorescence intensity, 2) Mean: Mean value and 3) Area: Size of the measured area.

### 2. Cloning of the exBiFC plasmids

### 2.1 Cloning of the exBiFC starting plasmids

Initially the starting plasmids pCMV-mRFP-YC and pCMV-CFP-YN were exemplary cloned in the cloning procedure.

### 2.1.1 Cloning of pCMV-mRFP-YC

### 2.1.1.1 pCMV-mRFP

The pCMV-mRFP plasmid, the first exBiFC starting plasmid, was cloned via the plasmid pCMV-mRFP. Therefor, pSV40-mRFP was used as matrix for amplification of mRFP1, the monomeric red fluorescent protein, via PCR. MRFP1 was amplified with the overlapping primers 48600 and 48604, simultaneously introducing restriction sites. Accordingly the restriction sites SACII and Nhel were added at the 5'end and coupled to the amplified mRFP via a linker. Subsequently a BamHI restriction site, a STOP codon and a Xbal restriction site were inserted. The inserted restriction sites were used for further cloning steps. For control purposes the amplification product was applied to an agarose gel, cut therefrom and purified. Subsequently the purified PCR product was cloned into the TOPO® vector, and this vector was transformed and plated. Three white colonies were isolated and a pre-culture was inoculated. From the two grown cultures the plasmid DNA was isolated according to the peQLab protocol. In order to control as to whether the amplification product is present in the TOPO® vector an analytic restriction digest of the plasmid was performed with the restriction enzymes Sacll and Xbal. Dependent on the orientation of the amplification product in the TOPO® vector three bands were expected, located at positions 3862, 768 and 6 (not recognisable in the gel) for a positive (+) orientation and at positions 3859, 774 and 58 (not recognisable in the gel) for a negative (-) orientation. The orientation of the amplification product in the TOPO® vector usually is not significant since the desired fragment will be cut and cloned into the final vector. One of two control plasmids showed the correct band pattern in the gel and accordingly integrated the amplification product. This clone was sequenced in order to verify the proper mRFP-sequence. Comparing the sequence and the expected sequence revealed a silent mutation at position 106 in mRFP (CGC -> CGT), having, however, no influence on the coding amino acid. Furthermore, one G was missing in the Xbal restriction site (852), destroying the same. Since the amplification product was present in a (+) orientation in the TOPO® vector and the vector also contained a Xbal restriction site at position 913, it could be used for the further cloning steps.

Afterwards a preparative restriction digest of the sequence plasmid-mini-preparation of the TOPO® -mRFP was performed with the restriction enzymes Sacll and Xbal. The 829 bp long fragment containing the mRFP was excised from the gel and purified. Simultaneously the target vector pCMV143/oligomaster was digested with the same restriction enzymes (Sacll and Xbal), excising the GFP contained therein. The cut vector was subsequently isolated from the gel and purified. Vector and insert were ligated afterwards. The two different restriction sites provided the requirement for inserting the insert in the correct orientation into the final vector. After ligation a transformation in XL10 supercompetent cells was carried out. The successful ligation was controlled by carrying out an analytical restriction digest with the restriction enzymes BsrGl and Kpnl. Seven of 12 controlled clones showed the expected bands of 5119 bp and 1138 bp in the agarose gel. A positive pCMV-mRFP clone was selected, a large culture inoculated starting from the pre-culture and a plasmid-maxi-preparation was performed.

### 2.1.1.2 pCMV-mRFP-YC

In order to clone the complete starting vector pCMV-mRFP-YC the amplification of the YFP C-terminus (YC: YFP-C-terminus; AS 155-249) was necessary. Therefor, a matrix was prepared by carrying out a PCR with the overlapping primers 48598 and 48602 and a plasmid p3'SS-Venusdimerlacl. Since this PCR only amplifies the C-terminus of Venus (amino acid 155 to 238) only the part is amplified being identical with the original form of YFP. A BamHI restriction site is introduced to the 5'end of the amplification product via PCR and a Xbal restriction site is introduced into the 3' side.

The amplification product was applied to an agarose gel, cut therefrom and purified. Afterwards the fragment was cloned into the TOPO® vector. After the blue-white selection six white clones were selected and a pre-culture of each one was inoculated. A plasmid-mini-preparation (according to peQLab) was prepared for the cultures. Subsequently an analytical restriction digest was performed with BAMHI and Xbal in order to identify proper clones. Four bands were expected in the gel, in particular 3814, 314, 61 and 45 (wherein the small bands were not recognisable in the gel) for a positive and a negative orientation of the YC fragment in the TOPO® vector. Four of six clones showed this band pattern in the agarose gel. According to the sequencing reaction the depicted clone showed several mutations and deletions. A large culture was inoculated starting from the pre-culture of this clone and the plasmid DNA was isolated via a plasmid-maxi-preparation. The above-mentioned mutations and deletions were removed by site-directed mutagenesis. Therefor, primers were used being localised in the mutated region but coding for the correct sequence. The plasmid-maxi-preparation from TOPO® -YC was used as a matrix. The mutagenesis reaction was performed with the primers 49142 and 49143. Subsequently 12 colonies were picked and a pre-culture of each was inoculated. A plasmid-mini-preparation (Qiagen) and an analytic restriction digest was performed for each of the 12 cultures with BamHI and Xbal. Each clone displayed the above-mentioned band pattern in the agarose gel. A proper plasmid-mini-preparation (peQLab) was prepared from a selected clone starting from the pre-culture. According to the sequencing the clone was error-free. Afterwards, a large culture of this TOPO® -YC clone was inoculated and the plasmid DNA was isolated via plasmid-maxi-preparation. Subsequently TOPO® -YC was preparatively digested with the restriction enzymes BAMHI and Xbal. The expected band of 314 bp was excised from the agarose gel and isolated.

The final cloning of pCMV-mRFP-YC was performed afterwards. Therefor, also pCMV-mRFP was preparatively digested with BamHI and Xbal. The vector of pCMV-mRFP was excised from the agarose gel and purified. After estimating the isolated DNA quantities in the agarose gel the vector and insert were ligated. The ligation reaction was transformed into bacteria and pre-cultures were inoculated afterwards from six selected colonies. The plasmid DNA was isolated via mini-plasmid-preparation from these colonies and controlled by performing an analytical restriction digest with SACII and Xbal. A positive pCMV-mRFP-YC clone was selected and a large culture was inoculated. Therefrom, plasmid DNA was isolated via plasmid-mini-preparation. In order to verify the preparation it was again analytically digested with Sacll and Xbal. In the agarose gel expected bands were visible at 5428 and 1066 bp. By using cloning a linker of 29 amino acids (SEQ ID NO: 1: GAGATSSGEGSTGSGSTSGSGKPGSGEGS) was inserted between the ORF of mRFP and YC. The linker was modified according to Whitlow et al. (1993, An improved linker for single-chain Fv with reduced aggregation and enhanced proteolytic stability. Protein Engineering Vol. 6, 989-995). The linker is thus resistant against proteolysis and diminishes aggregation.

### 2.1.2 Cloning of pCMV-CFP-YN

### 2.1.2.1 pCMV-CFP

pCMV-CFP was cloned according to the same principle as pCMV-mRFP. Therefor, a PCR was performed with the overlapping primers 48599 and 48603 in order to amplify CFP. The plasmid pSV40cenp-b-CFP was used as a matrix in this process. Also in this PCR the primers introduced a Sacll and a Nhel restriction site at the 3' end which were coupled to CFP via a linker. Subsequently a BamHI restriction site, a STOP codon and a Xbal restriction site were attached to the CFP. This amplification product was also applied to a gel, isolated therefrom and purified. The product again was cloned into the TOPO® vector. Six colonies were selected according to the blue-white selection and pre-cultures were inoculated. Plasmid-mini-preparations were prepared from the three grown cultures. An analytic restriction digest was performed with Sacll and Xbal in order to verify as to whether the PCR product was integrated into the TOPO® vector. For a positive orientation the three expected bands in the agarose gel were located at 3862, 795 and 61 bp and for negative orientation the bands were located at 3859, 801 and 58. In each case the smallest fragments are no more visible in the gel. The sequencing of the TOPO® -CFP clone showing the correct band pattern revealed an error-free product. A large culture was inoculated from the pre-culture of the positive clone and DNA was isolated via a plasmid-maxi-preparation. A preparative digest of the plasmid DNA of the TOPO® -CFP clone was prepared with the restriction enzyme Sacll and Xbal. The fragment of 795 bp containing CFP was excised from the gel and purified. The fragment (insert) and the vector pCMV143 (compare cloning of pCMV-mRFP), digested with the same enzymes, were ligated. The ligation reaction was transformed into bacteria. Twelve of the grown colonies were selected and a pre-culture of each one was inoculated. The plasmids were again isolated via a plasmid-mini-preparation. Successful ligation was controlled via an analytical restriction digest. Thereby the clones were digested with the restriction enzyme BsrGI, wherein two bands were expected having a size of 4758 and 1465 bp. All tested clones showed the band pattern expected for the pCMV-CFP plasmid. One clone was selected and a large culture was inoculated starting from its pre-culture. The plasmid DNA was isolated subsequently via a plasmid-maxi-preparation.

### 2.1.2.2 pCMV-CFP-YN

Initially, cloning of the second exBiFC starting plasmid was performed via the amplification of the N-terminus of YFP (YN). Plasmid pCMVRevlinkYFP was used as a matrix for YN. On the 5' side of YN a BamHI restriction site and on the 3' side a Xbal restriction site were introduced by using the overlapping primers 48597 and 48601. After performing the PCR the amplification product (~ 511 bp) was applied to a gel, cut therefrom and purified. Afterwards the product was cloned into the TOPO® vector. In total twelve white colonies were selected and pre-cultures were inoculated for each one of these. Plasmid DNA was isolated via plasmid-mini-preparation from these cultures and controlled with BamHI and Xbal via an analytical restriction digest. The expected band pattern in the agarose gel contains four bands of 3814, 500, 61 and 45 bp. After sequencing a large culture was inoculated starting from an error-free clone and plasmid DNA was isolated via a plasmid-maxi-preparation. A preparative digest of the TOPO® -YN plasmid was performed with BamHI and Xbal, after which the fragment of approximately 500 bp was cut from the gel and purified.

Simultaneously pCMV-CFP was preparatively digested with BamHI and Xbal and the cut vector was excised from the gel and purified. After estimating the molar ratios of vector and insert in the agarose gel ligation of the cut pCMV-CFP vector and the YN-insert was performed. The reaction was transformed in XL10 supercompetent cells. Afterwards six colonies were selected and plasmid DNA was prepared via plasmid-mini-preparation from the inoculated pre-cultures. The clones were verified by analytical restriction digest with Sacll and Xbal, wherein the band pattern had to display two bands of 5428 and 1297 bp in the agarose gel. A positive clone was selected, a large culture inoculated starting from its pre-culture and plasmid DNA was isolated via a plasmid-maxi-preparation. In order to control the result an analytical restriction digest was performed with Sacll and Xbal, upon which the expected band pattern of pCMV-CFP-YN was observed in the gel. By using cloning a linker of 16 amino acids was inserted between the ORF of CFP and YN (SEQ ID NO: 2: SEGEGSTGSGSTSGSG). The linker was also modified according to Whitlow et al. (1993, An improved linker for single-chain Fv with reduced aggregation and enhanced proteolytic stability. Protein Engineering Vol. 6, 989-995). The linker is thus stable against proteolysis and diminishes aggregation.

### 2.1.3 Cloning of the protein to be tested

In order to control the functionality of the system according to the present invention different proteins to be tested were cloned. For this proteins of the HIVI-gene were selected. In the following POI (= Protein of Interest) shall refer to the protein to be tested.

### 2.1.3.1 Cloning of Rev and Rev mutants

### 2.1.3.1.1 sRev

Initially sRev (synthetic Rev, according to Mermer, B et al., 1990) was amplified. PS40-mut-CsRev-YFP_inbig was used as a matrix and the overlapping primers were 37443 5'-sacll-Rev and 3744 3'mutant-Rev-Nhel, introducing the restriction sites for Sacll and Nhel. After PCR the amplification product was applied to an agarose gel and the expected bands of 380 bp was excised from the gel and purified. After the subsequent TOPO® -cloning step 3 colonies were selected, two of which were grown in the pre-culture. Plasmid DNA was prepared therefrom via a plasmid-mini-preparation and tested via an analytical restriction digest with Sacll and Xbal. In one TOPO® -sRev clone the expected bands of 3876 and 459 bp were observed in the agarose gel. The sequencing reveals an error-free sRev. A large culture of this clone was inoculated and DNA was isolated via a plasmid-maxi-preparation. This TOPO® -sRev vector was subsequently subjected to a preparative digest with Sacll and Nhel, the expected fragment of 380 bp was excised from the gel and purified.

### 2.1.3.1.2 RevM5

The plasmid pCRevM5sg143 was used as a matrix for Rev M5, the amplification of which was performed via PCR with the overlapping primers 37443 5'-Sacll-Rev and 3744 3'mutant-Rev-Nhel. The amplified fragment of 377 bp was excised from the gel and purified. The fragment was cloned into the TOPO® vector, upon which four of the grown white colonies were selected and pre-cultures were inoculated from each of these. Plasmid DNA isolation was performed via mini-preparation according to the peQLab protocol. The RevM5 sequence of a clone was sequenced, showing the correct band pattern of 3946 and 377 bp in the agaros egel after an analytical restriction digest with Sacll and Xbal. The TOPO® -RevM5 clone was shown to be error-free, upon which a large culture was inoculated. Plasmid DNA was isolated via a plasmid-maxi-preparation. The DNA was digested with the restriction enzymes Sacll and Nhel, upon which the generated fragment of 377 bp was excised from the gel and purified.

### 2.1.3.1.3 RevM10BL

The frahment Sacll-RevM10BL-Nhel was prepared according to the preceding fragments. For this purpose the encoding gene was cloned into an expression vector, RevM10BL was expressed and purified. The gene product was cut with the restriction enzymes Sacll and Nhel, applied to a gel, excised from the gel and purified.

### 2.1.3.1.4 RevM5M10BL

Plasmid pCRevM5+M10(Kombi)sg143 was used as a matrix for RevM5M10BL in an amplification reaction by using a PCR using the overlapping primers 37443 5'-Sacll-Rev and 3744 3'mutant-Rev-Nhel. The amplified band of 392 bp was subsequently excised from the agarose gel and purified. After cloning the fragment into the TOPO® vector four white colonies were selected, a pre-culture was inoculated and the DNA was isolated via a plasmid-mini-preparation. The clone was sequenced, showing a band pattern of 3861 and 471 bp in the agarose gel after an analytical restriction digest with Sacll and Xbal. Sequencing of this TOPO® -RevM5M10BL clone revealed no error in the RevM5M10BL sequence. Accordingly a large culture was inoculated and the plasmid DNA was isolated via a plasmid-maxi-preparation. The DNA was digested with the restriction enzymes Sacll and Nhel. Thereafter the desired band of 392 bp was excised from the gel and purified.

### 2.1.3.1.5 sRev(noNLS)

The amplification was performed via PCR using the overlapping primers 37443 5'-Sacll-Rev and 3744 3'mutant-Rev-Nhel and the plasmid pCsRev(noNLS)sg143 as a matrix. The revealed amplification product of 359 bp was excised from the gel and purified. Subsequently the fragment was cloned into the TOPO® vector. Four white colonies were selected, pre-cultures were inoculated and the plasmid DNA was isolated from the bacteria via a mini-preparation. In the agarose gel two clones showed the expected band pattern of 3867 and 438 after an analytical restriction digest with Sacll and Xbal. The sRev(noNLS)-sequence of a clone was sequenced and a large culture was inoculated starting from the error-free TOPO® -sRev(noNLS) clone and the DNA was isolated via a plasmid-maxi-preparation. Afterwards, the clone was digested preparatively with Sacll and Nhel, whereafter the desired fragment of 358 bp was excised from the gel and purified. The fragment was now available for further cloning steps.

### 2.1.3.1.6 RevPAAAA

The plasmid pCRevPAAAAsg143 was used for amplification by using a PCR using the overlapping primers 37443 5'-Sacll-Rev and 3744 3'mutant-Rev-Nhel as a matrix. The amplification product was applied to an agarose gel and the desired band of 380 bp was excised from the gel and purified. After the subsequent TOPO cloning step three white colonies were selected and pre-cultures were inoculated. Plasmid DNA was isolated via mini-preparation from these pre-cultures and analytically digested with Sacll and Xbal. Thereby two clones revealed the expected band pattern of 3867 and 459 bp, one of which was selected and sequenced. The sequencing reaction revealed that the BamHI restriction site in sRevPAAAA was deleted by a silent point mutation from ATC to ATT, wherein this mutation was already present in the starting plasmid and intended. This mutation does not have any impact for the further cloning steps. Starting from this TOPO® -sRevPAAAA clone a large culture was inoculated from the pre-culture and the plasmid DNA was isolated via a maxi-preparation. A preparative restriction digest was prepared with Sacll and Nhel, upon which the desired band was excised from the geld and purified. The fragment showed a length of 380 bp.

### 2.1.3.1.7 Rev54-116

The desired fragment Rev54-116 was excised via a preparative digest with Sacll and Nhel from the plasmid pCMVBspEIRev54-116-GFP. The desired band of 245 bp was excised from the gel, purified and used for the further cloning steps.

### 2.1.3.2 Cloning of CRM1

In the plasmid pCCrm1-sg143 CRM1 was present in a suitable form. It was digested with the restriction enzyme Nhel. The created 3225 bp fragment could directly be used for further cloning steps.

### 2.1.3.3 Cloning of Importinβ

Importinβ was amplified by using the PCR with the overlapping primers 49969 and 49970 and the plasmid pChlmpβ as a matrix. Thereby a Sacll restriction site was attached to the 5' end and a AvrII restriction site was attached to the 3' end of the amplification product. The amplification product was applied to an agarose gel and the desired band of 2671 bp was excised from the gel and purified. After cloning of the fragment into the TOPO® vector four white colonies were selected and a pre-culture was inoculated from each of those. The plasmid DNA resulting therefrom was analytically digested with Sacll and Nhel. All of the four clones showed the expected bands of 5992 and 586 bp. One clone was selected and the Importinβ sequence was sequenced with the primers 49966, 49967 and 49968. The sequencing revealed a silent mutation from TAC to TAT (Tyr544), as well as a mitation from alanine to valine at position 628. The further cloning step was performed with Importinβ mutated as indicated at this position. The plasmid-mini-preparation of the TOPO®⁻Importinβ(A628V) was preparatively digested with Sacll and Avrll. Since Acrll is an isochisomer of Nhel the ligation of an Avrll and a Nhel sticky end in a further cloning step can be carried out without any problems. The resulting fragment of 2654 bp was excised from the gel, purified and used for further cloning steps.

### 2.1.3.4 Cloning of Tat

Tat was amplified by using a PCR with the overlapping primers 43132 and 43133 as well with the plasmid pF25Tat-GFP. By using this reaction a Sacll restriction site was added to the 5' end and a Nhel restriction site was added to the 3' end. The amplification product was applied to a gel and the expected band of 300 bp was excised from the gel and purified. After cloning into the TOPO® vector six white colonies were selected and from each of those pre-cultures were inoculated. From such pre-cultures plasmid DNA was isolated and analytically digested with BamHI and Xbal. The Tat-sequence of a clone was sequenced showing the band pattern of 3814 and 395 in an agarose gel. The mini-preparation of the TOPO® -Tat clone containing an error-free sequence was preparatively digested with the restriction enzymes Sacll and Nhel. The resulting fragment of 277 bp was excised from the gel, purified and used for further cloning steps.

### 2.1.3.5 Cloning of Nef

The amplification of Nef was performed by using a PCR with the primers 43130 and 43131 using the plasmid pCNefsg25GFP as a matrix. Analogous to Tat the restriction sites Sacll and Nhel were added on both sides of the amplification product. The desired band of 648 bp was excised from the agarose gel and purified. After the TOPO®⁻cloning step six white colonies were also selected and the plasmid DNA was isolated via mini-preparation. The analytic restriction digest with BamHI and Xbal revealed the desired band pattern of 3814 and 774 bp for all clones in the agarose gel. The Nef-sequence of a selected clone was sequenced and shown to be error-free. The plasmid-mini-preparation of this TOPO® -Nef clone was also preparatively digested with the restriction enzymes Sacll and Nhel, the resulting fragment of 635 bp was excised from the gel, purified and used for further cloning steps.

### 2.2 Cloning of the exBiFC final plasmids

For cloning of the final exBiFC plasmids each of the starting plasmids pCMV-mRFP-YC and pCMV-CFP-YN were preparatively digested with the restriction enzymes Sacll and Nhel. The cut vector was excised from the geld and purified, upon which the desired band for pCMV-CFP-YN was observed at 6690 and for pCMV-mRFP-YC at 6478 bp. For the cloning step of CRM1 the vector pCMV-mRFP-YC was subjected to a preparative digest solely with Nhel and subsequently dephosphorylated. The cut plasmids were now used as vectors for the ligation with the corresponding fragments of the POIs for cloning the exBiFC plasmids (see Figs. 2 to 5 and 17 to 24).

### 2.2.1 pCMV-sRev-CFP-YN

By using an agarose gel the quantities of the vectors pCMV-CFP-YN digested with the restriction enzymes of SACII and Nhel and the inserts sRev digested with the same enzymes were controlled and appropriate quantities of vector and insert were used for the ligation reaction. The ligation reaction was subsequently transformed in bacteria and plated. Four colonies were selected and pre-cultures were inoculated. Thereafter plasmid DNA was isolated from bacteria according to the peQLab protocol. The success of ligation was verified by using an analytical restriction digest with BamHI. The expected band pattern in the agarose gel shows bands of 6120 and 950 bp. One of the positive pCMV-sRev-CFP-Yn clones was selected, a large culture was inoculated and a plasmid-maxi-preparation was performed. This clone was now available for further transfection experiments.

### 2.2.2 pCMV-RevM5-CFP-YN

The cloning of this plasmid was performed in the same way as the preparation of pCMV-sRev-CFP-YN. The expected bands in the agarose gel at the analytic restriction digest were identical. PCMV-RevM5-CFP-YN was available for further experiments after plasmid-maxi-preparation.

### 2.2.3 pCMV-RevM10BL-CFP-YN

Also RevM10BL was cloned in the same way as the two before-mentioned plasmids. The expected band in the agarose gel after the analytical restriction digest were 6120 and 962. pCMV-RevM10BL-CFP-YN was also now available for further experiments after plasmid-maxi-preparation.

### 2.2.4 pCMV-RevM5M10-CFP-YN

The cloning of pCMV-RevM5M10-CFP-YN was prepared exactly following the same scheme as shown for pCMV-REVM10BL-CFP-YN.

### 2.2.5 pCMV-RevPAAAA-CFP-YN

The cloning of this plasmid was also performed according to the same principle as the preceding plasmids, the analytic restriction digest was, however, performed with Sacll and BamHI, upon which the expected pattern in the Gel revealed bands of 5928 and 1142 bp. After a plasmid-maxi-preparation pCMV-RevPAAAA-CFP-YN was also available for further experiments.

### 2.2.6 pCMV-Rev(noNLS)-CFP-YN

The cloning of Rev(noNLS) also followed the same principle. The expected bands after the analytical restriction digest with BamHI were 6099 and 950 bp. Also pCMV-Rev(noNLS)-CFP-YN was available after a plasmid-maxi.preparation.

### 2.2.7 pCMV-Tat-CFP-YN

The cloning of Tat was performed according to the afore-mentioned scheme, the ligation was, however, controlled by using an analytic restriction digest with Sacll and Xbal. In this digest the band pattern of 5428 and 1540 were obtained in the agarose gel. pCMV-Tat-CFP-YN was available for further experiments after the plasmid-maxi-preparation.

### 2.2.8 pCMV-sRev-mRFP-YC

The cloning steps were performed as indicated above. The analytic restriction digest for controlling the success of ligation was performed again with the restriction enzyme BamHI. The band in the agarose gel obtained therefrom displayed a length of 5934 and 923 bp. The pCMV-sRev-mRFP-YC was also available for further experiments after plasmid-maxi-preparation.

### 2.2.9 pCMV-Rev(noNLS)-mRFP-YC

The cloning steps were performed according to the above-mentioned scheme following a control by using an analytic restriction digest with BamHI. The expected bands of 5913 and 923 bp were observed for all tested clones. One clone was selected and a plasmid-maxi-preparation was prepared. pCMV-Rev(noNLS)-mRFP-YC was now also available for further experiments.

### 2.2.10 pCMV-RevPAAA-mRFP-YC

These cloning steps were performed similar to the cloning steps shown above. However, the successful cloning step was controlled by using an analytic restriction digest with Sacll and Xbal. The expected bands of 5428 and 1429 bp were observed for all tested clones. After selection and plasmid-maxi-preparation of one clone pCMV-RevPAAAA-mRFP-YC was available for further experiments.

### 2.2.11 pCMV-Rev54-116-mRFP-YC

The cloning of Rev54-116 into the final vector was performed according to the above-discussed scheme. An analytic restriction digest with BamHI revealed the expected bands of 5790 and 932 bp in the agarose gel. pCMV-Rev54-116-mRFP-YC was available for further experiments after a plasmid-maxi-preparation.

### 2.2.12 pCMV-Tat-mRFP-YC

The cloning reactions were prepared as mentioned above, whereby the analytic restriction digest was performed with the restriction enzymes Sacll and Xbal. The expected bands of 5428 and 1327 bp were observed for all tested clones in the agarose gel. Accordingly pFED-Tat-mRFP-YC was also available after plasmid-maxi-preparation of a selected clone for further experiments.

### 2.2.13 pCMV-Nef-mRFP-YC

Analogous to the above-mentioned proceedings Nef was ligated into the final vector. The analytic restriction digest was performed according to pCMV-Tat-mRFP-YC with the restriction enzymes Sacll and Xbal. All tested clones showed the resulting bands of 5428 and 1685 bp. One selected clone from pCMV-Nef-mRFP-YC was available for further experiments after plasmid-maxi-preparation.

### 2.2.14 pCMV-CRM1-mRFP-YC

The cloning of CRM1 was performed similar to the above.mentioned principle. However, for further cloning pCMV-mRFP-YC was solely digested with Nhel, since the CRM1 fragment also contained Nhel restriction sites on both sides. Using the analytic restriction digest with Xhol and EcoRV it was not only controlled as to whether ligation was successful but also as to whether CRM1 was integrated with its right orientation. In the case of a positive orientation one would expect a pattern of 8873 and 846 bp in the agarose gel and bands of 5792 and 3927 bp in the case of a negative orientation. Of twelve tested clones four contained CRM1 in positive orientation. One of these was selected for preparing a plasmid-maxi-preparation. pCMV-CRM1-mRFP-YC was now also available for further experiments.

### 2.2.15 pCMV-Importinβ(A628V)-mRFP-YC

The cloning of the Importinβ(A628V)-insert with the ends Sacll and Avrl in a vector cut with Sacll ad Nhel is possible, since Avrl and Nhel are isoschizomeric restriction sites. For the purpose of the cloning step both overlapping ends can be assembled and ligated without any problem. However, subsequently neither a restriction site for Avrl nor for Nhel is present anymore. Using the restriction enzymes BamHI and Nhel the clones were tested with respect to a successful ligation. The obtained band pattern of 6328 and 2803 bp was observed in the agarose gel for five of six tested clones. One of those was selected and its plasmid-maxi-preparation was used for further experiments.

### 3. Transfection Experiments

### 3.1 Determination of the background fluorescence of the system

For the BiFC-system it was assumed that the N-terminal and the C-terminal domains of YFP being expressed in the cells do not exhibit a fluorescence on their own In order to verify as to whether this assumption also applies to the method according to the present invention firstly the starting plasmids pCMV-CFP-YN and pCMV-mRFP-YC were solely transfected in HeLa cells: In the following the results are described shortly. For illustration purposes a representative image is shown for each transfection (see Figs. 9 to 12).

### 3.1.2 pCMV-CFP-YN

In this multi-channel image of the transfection with pCMV-CSP-YN only one signal in the CFP channel can be observed. In the YFP channel no fluorescence is detected (see Fig. 3A).

### 3.1.3 pCMV-mRFP-YC

Also in this case only one fluorescence signal is observed in the mRFP channel and not in the YFP channel. Apparently both halves of YFP are on their own not able to generate a fluorescence signal in the YFP channel in the method according to the present invention. Both YFP halves are also not activated such as to emit a fluorescence signal by forming of the respective fused fluorochrome (see Figure 3B). The exBiFC starting plasmids pCMV-CFP-YN and pCMV-mRFP-YC were initially cotransfected for the measurement in order to determine the background fluorescence.

### 3.1.4 pCMV-CFP-YN + pCMV-mRFP-YC

For the first time a signal is observed in the YFP channel. A quantification of the fluorescence intensities is also necessary, which must also be put into relation to each other. The fluorescence observed in the present case represents the expected background fluorescence resulting from an unspecific interaction of the resulting proteins of the two transfected starting plasmids of exBiFC.

### 3.2 Localisation of the single exBiFC-plasmids with their POIs

The different exBiFC final plasmids were each transfected in HeLa cells in order to control the phenotype of the localisaton of all POIs. For this purpose initially different amounts of plasmid DNA were transfected and the cells were observed and photographed at a different time period in the Epifluorescence microscope Cellobserver. Following several trials a transfection amount of 150 ng plasmid DNA proved to be optimal for all Rev, Rev-mutants, Tat and Nef plasmids, as well as 1000 ng plasmid DNA of CRM1 and Importinβ(A62V) plasmids, since the expression of the different proteins was similar in its intensity. The images were performed after 48 hours. After single transfections the different fusion proteins showed the following localisation:

**Table 1: Localisation of the final plasmids with different POIs**

| Fusion protein | Predominant localisation |
|---|---|
| sRev-CFP-YN | in the nucleoli |
| sRev-mRFP-YC | in the nucleoli and cytoplasmic |
| RevM5-CFP-YN | cytoplasmic |
| RevM5-mRFP-YC | cytoplasmic |
| RevM10BL-CFP-YN | in the nucleoli |
| RevM10BL-mRFÜP-YC | in the nucleoli |
| RevM5M10BL-CFP-YN | in the nucleus, not in the nucleoli |
| RevM5M10BL-mRFP-YC | in the nucleus, not in the nucleoli |
| RevPAAAA-CFP-YN | in the nucleoli |
| RevPAAA-mRFP-YC | in the nucleoli |
| Rev(noNLS)-CFP-YN | in the cytoplasma |
| Rev(noNLS)-mRFP-YC | in the cytoplasma |
| Rev54-116-CFP-YN | in the cytoplasma |
| Rev54-116-mRFP-YC | in the cytoplasma |
| Tat-CFP-YN | in the nucleoli |
| Tat-mRFP-YC | in the nucleoli |
| Nef-CFP-YN | at the plasma membrane and the Golgi apparatus |
| Nef-mRFP-YC | at the plasma membrane and the Golgi apparatus |
| CRM1-CFP-YN | at the nuclear membrane |
| CRM1-mRFP-YC | at the nuclear membrane |
| Importin(A628V)-CFP-YN | at the nuclear membrane |
| Importin(A628V)-mRFP-YC | at the nuclear membrane |

Despite of sRev all shown localisations of the exBiFC final plasmids were as expected.

### 3.3 Dimerisation of sRev as the positive control

The cotransfection of sRev, forming multimers and as described in the following serves as an indication for a positive interaction of two proteins. Therein, sRev shows an untypical localisation of srev in connection with mRFP-YC. The interaction of both YFP-halves leads to a complex formation of sRev-mRFP-YFP-CFP-sRev in the cytoplasm. The extremely strong signal in the yellow channel shows that the method according to the present invention properly works and the images disclose a positive interaction of the protein sRev (see Figure 4).

### 3.4 Rev and Rev-mutants

In the following exemplary cotransfection experiments of sRev and different Rev-mutants were performed.

### 3.4.1 pCMV-sRev-CFP-YN + pCMV-Rev54-116-mRFP-YC

The interaction of the cytoplasmic localised Rev54-116-mRFP-YC also has an impact on the localisation of the exBiFC complexes in the present case. The nucleic localisation of the sRev protein usually occurring in HeLa cells is shifted to the cytoplasm due to the complex formation (see Figure 5A).

### 3.4.2 RevM10BL-CFP-YN + pCMV-sRev-mRFP-YC

RevM10BL-CFP-YN is localised in the nucleus, whereas sRev in connection with mRFP-YC, as already mentioned, also localises in the cytoplasm additionally to the nucleoli. A small YFP signal can be observed in the cytoplasm and the nucleoli (see Figure 5B).

### 3.4.3 pCMV-RevM5M10BL-CFP-YN + pCMV-sRev-mRFP-YC

After formation of the exBiFC complexes the nucleic localised RefM5M10BL-CFP-YN exhibits a more cytoplasmic localisation in connection with the sRev-mRFP-YC in the present case. The fluorescence in the YFP channel is apparently weak (see Figure 5C).

### 3.4.4 pCMV-sRev-CFP-YN + pCMV-RevPAAAA-mRFP-YC

sRev-CFP-YN as well as RevPAAAA-mRFP-YC exhibits both a nucleic localisation, which can be clearly observed in the CFP and mRFP channel. In the cytoplasm and the nucleoli a weak YFP signal can be observed (see Figure 5D).

### 3.4.5 pCMV-Rev-CFP-YN + pCMV-sRev-mRFP-YC

RevM5-CFP-YN localises cytoplasmic and sRev-mRFP-YC, as already mentioned above, localises in the nucleoli as well as in the cytoplasm. A very weak fluorescence in the YFP channel is observed in the cytoplasm (see Figure 5E).

### 3.4.6 pCMV-RevM10BL-CFP-YN + pCMV-RevPAAAA-mRFP-YC

Along with RevM10BL-CFP-YN and RevPAAAA-mRFP-YC again two proteins are observed showing a nucleic localisation. The strong fluorescence signal in the nucleoli in the yellow channel provides a strong indication for the interaction of the proteins (see Figure 5F).

### 3.4.7 pCMV-RevM5-CFP-YN + pCMV-Rev54-116-mRFP-YC

As mentioned above RevM5-CFP-YN localises in the cytoplasm as well as Rev54-116-mRFP-YC. In the cytoplasm of expressed cells a YFP signal can be observed (see Figure 5G).

### 3.5 Rev, Tat and Nef

To provide an additional exemplary application of the method according to the present invention the interactions of the viral proteins sRev, Tat and Nef were determined, since their interaction has been assumed in part (see Figures 6A-C).

### 3.5.1 pCMV-sRev-CFP-YN + pCMV-Nef-mRFP-YC

sRev-CFP-YN localises upon formation of the exBiFC complex with Nef-mRFP-YC in the nucleoli and in the cytoplasm. Based on the weak signal in the YFP channel it can be deduced that both proteins do not interact with each other (see Figure 6A).

### 3.5.2 pCMV-Tat-CFP-YN + pCMV-sRev-mRFP-YC

Also for Tat-CFP-YN being localised in the nucleoli upon single transfection, the signal of the formed exBiFC complex is very weak in the YFP channel upon cotransfection with sRev-mRFP-YC. Accordingly, apparently no interaction takes place in the present case (see Figure 6B).

### 3.5.3 pCMV-Tat-CFP-YN + pCMV-Nef-mRFP-YC

The cotransfection of Tat and Nef confirms the above described localisations of both proteins (see Figure 6C).

### 3.6 Rev and CRM1/Exportin

Subsequently interactions from Rev and different mutants with the cellular export protein CRM1/Exportin were determined (see Figures 7A-E).

### 3.6.1 pCMV-sRev-CFP-YN + pCMV-CRM1-mRFP-YC

Initially, sRev-CFP-Yn was transfected with CRM1-mRFP-YC. Upon formation of the exBiFC complex sRev-CFP-YN exhibited a cytoplasmic localisation, whreas CRM1-mRFP-YC localised at the nuclear membrane. The signal in the YFP channel is apparently very strong (see Figure 7A).

### 3.6.2 pCMV-RevM10BL-CFP-YN + pCMV-CRM1-mRFP-YC

RevM10BL localises in the nucleoli and CRM1 at the nuclear membrane. The localisation of both proteins clearly can be observed (see Figure 7B).

### 3.6.3 pCMV-RevM5M10BL-CFP-YN + pCMV-CRM1-mRFP-YC

RevM5M10BL-CFP-YN is not localised at the nucleoli. This can also clearly be observed in the CFP channel. Additionally, the annular localisation of CRM1-mRFP-YC an the nuclear membrane can be observed clearly (see Figure 7C).

### 3.6.4 pCMV-RevPAAAA-CFP-YN + pCMV-CRM1-mRFP-YC

The localisation of RevPAAAA-CFP-YN and CRM1-mRFP-YC can clearly be observed:
RevPAAAA-CFP-YN in the nucleoli and CRM1-mRFP-YC at the nuclear membrane (see Figure 7D).

### 3.6.5 pCMV-Rev(noNLS)-CFP-YN + pCMV-CRM1-mRFP-YC

Likewise as seen in the before-mentioned transfections the protein localisation is also characteristic in the present case. Rev(noNLS)-CFP-YN is localise din the cytoplasm and CRM1-mRFP-YC is localised at the nuclear membrane (see Figure 7E).

### 3.7 Rev and Importinβ(A628V)

In the following the last two cotransfections with the A628V mutant of the cellular import-factor Importinβ are disclosed. The localisations of sRev-CFP-YN (nucleic) as well as of Importinβ(A628V)-mRFP-YC (nuclear membrane) clearly can be observed. The YFP-fluorescence is relatively weak (see Figure 8A).

### 3.8 pCMV-Tat-CFP-YN + pCMV-Importinβ(A628V)-mRFP-YC

The localisations of Tat-CFP-YN (nucleic) as well as of Importinβ(A628V)-mRFP-YC (nuclear membrane) can clearly be observed in the present case. The signal in the YFP channel is apparently stronger and cytoplasmic (see Figure 8B).

### Advantages of the Invention:

The methods according to the present invention for determining protein-protein-interactions provide transfection/expression and localization information by using inventive fusion proteins. These inventive fusion proteins are provided by fusing additional fluorochromes to the protein to be tested (component (a)). These fluorochromes (component (b)) allow the protein to be trapped in the cell and (components (c) and (c')) to analyze the interaction of components (a) and (a') of both fusion proteins A and A'. Therefore, both fusion proteins may be localised even if no interaction occurs. However, if interaction occurs, a third fluorescence signal indicates the interaction of components (a) and (a') of both fusion proteins. Accordingly, the method according to the present invention is superior over prior art methods due to additional information about transfection/expression of inventive fusion proteins.

Another embodiment of the present invention refers to an effective method for screening modulators, i.e. inhibitors or enhancers. Therein, the inventive fusion proteins are efficiently used to detect modulation of interactions of proteins to be tested in living cells and thus to screen test compounds for their modulating effect.

Advantageously, the inventive fusion proteins can also be used to detect a cell comprising unknown protein(s) that interact with a known protein. By this method, libraries containing randomly selected sequences encoding proteins or protein fragments may be screened for their interaction profile.

## Claims

1. Fusion protein A, containing components:
(a) protein sequence of a protein to be tested,
(b) fluorochrome group, and
(c) the N-terminal or C-terminal portion of a fluorochrome protein.

2. Fusion protein A according to claim 1, **characterized in that** components (a), (b) and (c) are assembled in any conceivable order.

3. Fusion protein A according to claims 1 or 2, **characterized in that** fluorochrome group (b) is essentially not identical with the N-terminal or C-terminal portion of the fluorochrome protein (c).

4. Fusion protein A according to any of claims 1 to 3, **characterized in that** the fluorochrome group (b) is a fluorochrome protein or a linker labelled with a fluorescent dye.

5. Fusion protein A according to claim 4, **characterized in that** the fluorescent dye is selected from the hemicyanes comprising DY-630-NHS or DY-635-NHS, or from the coumarins, comprising aminocoumarin, 7-amino-4-methylcoumarin, 7-hydroxy-4-methylcoumarin, AMCA (aminomethylcoumarin), 15, IAEDANS, and hydroxycoumarin.

6. Fusion protein A according to any of claims 1 to 4, **characterized in that** fluorochrome group (b) or N-and C-terminal portions of fluorochrome protein (c) are selected from a group consisting of blue fluorescent protein (BFP), green fluorescent protein (GFP), photo activatable-GFP (PA-GFP), yellow shifted green fluorescent protein (Yellow GFP), yellow fluorescent protein (YFP), enhanced yellow fluorescent protein (EYFP), cyan fluorescent protein (CFP), enhanced cyan fluorescent protein (ECFP), monomeric red fluorescent protein (mRFP1), kindling fluorescent protein (KFP1), aequorin, autofluorescent proteins (AFPs), JRed, TurboGFP, PhiYFP and PhiYFP-m, tHc-Red (HcRed-Tandem), PS-CFP2 and KFP-Red.

7. Fusion protein A according to any of claims 1 to 6, **characterized in that** a linker is inserted between components (a) and (b) and/ (b) and (c).

8. Fusion protein A according to claim 7, **characterized in that** the linker comprises a length of 5-40 amino acids, preferably of 5-25 amino acids and more preferably of 5-20 amino acids.

9. Fusion protein A according to any of claims 7 to 8, **characterized in that** the linker is composed of at least 35% of the amino acid glycine.

10. Fusion protein A according to any of claims 7 to 9, **characterized in that** the linker is selected from SEQ ID NO: 1 or SEQ ID NO: 2.

11. Fusion protein A according to any of claims 1 to 10, **characterized in that** component (a) is a prokaryotic or an eukaryotic protein, preferably a viral, bacterial, plant, animal or human protein.

12. Nucleic acid N encoding a fusion protein A according to any of claims 1 to 11.

13. Vector V containing at least one nucleic acid N according to claim 12.

14. Vector V according to claim 13, **characterized in that** the vector contains at least two nucleic acids N and N' according to claim 12, encoding different fusion proteins A and A' according to any of claims 1-11, whereby components (a) and (a') of fusion proteins A and A' are not identical.

15. Vector V according to any of claims 13 to 14, **characterized in that** the vector additionally contains at least one regulation sequence being functionally linked to nucleic acid N.

16. Host cell C, containing at least one vector V according to any of claims 13 to 15.

17. Host cell C according to claim 16, **characterized in that** the host cell contains at least two vectors V and V' according to claim 13 or 15 encoding non-identical fusion proteins A and A'.

18. Vector library LV, containing vectors according to any of claims 13-15.

19. Host cell library LC, containing host cells according to any of claims 16-17.

20. Method for detecting protein-protein interactions, comprising the steps of:
(I) providing at least one vector V and V' according to any one of claims 13 to 15; and
(II1) transfecting a host cell C according to any one of claims 16 to 17 with vector V as provided by step (I), wherein vector V comprises at least two nucleic acids N and N' according to claim 12 encoding fusion proteins A and A' according to any of claims 1 to 11, wherein components (a) and (a') of fusion proteins A and A' are not identical; or
(II2) transfecting a host cell C according to any one of claims 16 to 17 with at least two vectors V and V' as provided by step (I), wherein each vector V and V' comprises a nucleic acid N or N' according to claim 12 encoding fusion proteins A and A' according to any of claims 1 to 11, wherein components (a) and (a') of fusion proteins A and A' are not identical; and
(III) detecting fluorescence signals of fluorochrome components (b)/(b') and/or (c)/(c') of fusion proteins A and A' expressed in the host cell.

21. Method according to claim 20, **characterized in that** at least three fluorescence signals are detected by step (III).

22. Method for screening modulators of protein-protein interactions comprising the steps of:
(I) providing at least one host cell C according to claims 16 to 17 containing or expressing a first and a second fusion protein A, A' according to claims 1 to 11;
(II) providing and adding a test compound; and
(III)detecting an altered fluorescence signal of fusion proteins A and A' expressed in host cells.

23. Method according to claim 22, **characterized in that** the modulator is an inhibitor or an enhancer of protein-protein-interactions.

24. Method for detecting interactions of a first protein with a second protein comprising following steps:
(I) generating a host cell library LC according to claim 19, whereby each host cell comprises:
(I1) a first fusion protein A according to claims 1 to 11; and
(12) a second fusion protein A' according to claims 1 to 11;
(II) screening the host cell library for host cells by detecting a fluorescence signal of the expressed first and/or second fusion proteins A and A'.

25. Method according to any of claims 20 to 24, **characterized in that** fluorescence is detected by using laser-induced fluorescence detection (LIF), laser-induced time-staggered fluorescence detection (LI2F), fluorescence lifetime imaging microscopy (FLIM), spectrophotometry, flow cytometry, or white fluid fluorescence spectroscopy.

26. Kit for screening modulators of protein-protein interactions comprising as constituents a vector V (constituent 1) according to claims 13 to 15, wherein vector V encodes at least two fusion proteins A and A' according to claims 1 to 11, or at least two vectors V and V' (constituent 1') according to claims 13 to 15, wherein each vector encodes just one fusion protein A or A' according to claims 1 to 11, and optionally instructions for use (constituent 2).

27. Kit for screening modulators of protein-protein interactions comprising as constituents a host cell C (constituent 1) according to claims 16 to 17, being transfected with at least one vector V according to claims 13 to 15, wherein vector V encodes at least two fusion proteins A and A' according to claims 1 to 11, or host cells C and C' (constituent 1') according to claims 16 to 17, each host cell being transfected with just one vector V or V' according to claims 13 to 15, respectively, wherein each vector V or V' encodes just one fusion protein A or A' according to claims 1 to 11, and optionally instructions for use (constituent 2).

28. Use of a fusion protein A according to any of claims 1 to 11, a nucleic acid N according to claim 12, a vector V according to any of claims 13 to 15, a host cell C according to any of claims 16 to 17 , a vector library LV according to claim 18, a host cell library LC according to claim 19, or kits according to claim 26 to 27 for determining protein-protein interactions, for screening modulators of protein-protein interactions, or for determining interactions of a first protein with a second protein.


SEQUENCE LISTING

<110> GSF - Forschungszentrum fuer Umwelt und Gesundheit, GmbH

<120> Fusion proteins and methods for determining protein-protein-
interactions in living cells and cell lysates, nucleic acids encoding these
fusion proteins, as well as vectors and kits containing these

<130> GS01P002EP

<160> 2

<170> PatentIn version 3.1

<210> 1
<211> 29
<212> PRT
<213> Artificial

<220>
<221> misc_feature
<223> Description of artificial sequence: linker sequence

<400> 1
Gly Ala Gly Ala Thr Ser Ser Gly Glu Gly Ser Thr Gly Ser Gly Ser
1               5                   10                  15

Thr Ser Gly Ser Gly Lys Pro Gly Ser Gly Glu Gly Ser
            20                  25

<210> 2
<211> 16
<212> PRT
<213> Artificial

<220>
<221> misc_feature
<223> Description of artificial sequence: linker sequence

<400> 2

Ser Glu Gly Glu Gly Ser Thr Gly Ser Gly Ser Thr Ser Gly Ser Gly
1               5                   10                  15